# EUROPEAN PATENT SPECIFICATION

(11) **EP 4 233 817 B1**
(45) Date of publication and mention of the grant of the patent: **07.10.2026**
(21) Application number: 23169690.7
(22) Date of filing: 11.05.2018
(51) Int. Cl.: A61F 13/476, A61F 13/56, A61F 13/472

(54) **FEMININE HYGIENE ARTICLE**
ARTIKEL FÜR DIE WEIBLICHE HYGIENE
ARTICLE D'HYGIÈNE FÉMININE

(30) Priority: 12.05.2017 US 201762505152 P
(43) Date of publication of application: 30.08.2023
(60) Divisional application: 26201769.2
(62) Document number(s) of the earlier application(s) in accordance with Art. 76 EPC: 18732992.5 / 3 621 571
(73) Proprietor: The Procter & Gamble Company, Cincinnati, OH 45202 (US)
(72) Inventor: VOHWINKEL, Henning, 65824 Schwalbach am Taunus (DE); DOMEIER, Wolfgang Werner Hans, 65824 Schwalbach am Taunus (DE); HEFELE, Rainer, 74564 Crailsheim (DE); LAVASH, Bruce William, 45202 Cincinnati (US); WENDT, Holger, 65824 Schwalbach am Taunus (DE)
(74) Representative: P&G Patent Germany

(56) References cited:
- EP-A1- 1 208 823
- EP-A1- 2 216 003
- EP-A1- 3 075 365
- JP-A- 2013 220 225
- US-A1- 2005 124 959
- US-A1- 2015 057 632

## Description

### FIELD OF THE INVENTION

The present application pertains to absorbent articles and more particularly to feminine hygiene articles.

### BACKGROUND OF THE INVENTION

Feminine pads have been used widely for several decades. These pads are generally utilized during menstruation to capture menses and wetness. In general, the user of feminine pads wants to feel clean and dry. Many advancements in feminine pad technology have attempted to improve one or both of these aspects. For example, wings were introduced to the feminine pads to reduce the likelihood of leakage and to help secure the pad to the panty of the wearer. Additionally, absorbent core materials have been introduced which can provide enhanced absorbent capacity in a relatively thin pad.

However, providing the user of feminine pads the desired experience is a bit more complex and requires more than the above advancements. The user of the feminine pad should be provided with an easy to discern application guide which ensures the proper placement /orientation of the feminine pad within the panty. If improper placement and/or orientation is utilized, the above advancements of wings and absorbent core materials will likely not provide the intended benefit. Additionally, the pad must be securely fastened to the panty. Without the secure fastening of the feminine pad to the panty, the feminine pad may become detached during use. And similar to improper placement and/or orientation, detachment can negate much of the functionality of the advancements described above.

Secure attachment of the feminine pad to the panty can generally include two aspects. First, proper attachment of the pad to the panty, and second, sustained attachment / coverage of the feminine pad to the panty. Unfortunately, without proper attachment, sustained attachment and coverage may be difficult to achieve. And, in some instances, proper attachment does not necessarily guarantee sustained attachment / coverage.

EP 3075365 A1 discloses an absorbent article with a wing-shaped flap. EP 2216003 A1 discloses a sanitary napkin having wing-like flaps that may be colored at their end portions. JP 2013-220225A discloses an absorbent article in which a wing is less likely to be broken at an unintended portion during a mounting operation. US 2015/0057632 A1 discloses asymmetrical absorbent articles having printed regions to facilitate individual placement of the absorbent article. US 2005/0124959 A1 discloses an absorbent article with asymmetric protective wing portions. EP 1208823 A1 discloses absorbent articles with asymmetric flaps. Based on the foregoing, it would be beneficial to provide a pad which provided easily discernable placement / orientation guides, facilitated attachment guides, and/or sustained attachment to the panty.

### SUMMARY OF THE INVENTION

The invention provides a feminine hygiene article according to the claims.

The feminine hygiene article of the present invention can provide the user with an intuitive application guide which facilitates the identification of proper orientation of the pad. In some forms, the feminine hygiene article of the present invention can provide the user with an intuitive application guide which facilitates proper application of the pad to a panty. And, in some forms, the feminine hygiene article of the present invention can provide sustained coverage of the feminine hygiene article to the panty.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings described below.
Figure 1A is a representation of a feminine pad outside the subject-matter of the claims.
Figure 1B is an illustration of a first end of the feminine pad of Figure 1A.
Figure 1C is an illustration of a second end of the feminine pad of Figure 1A.
Figure 1D is an illustration of an intermediate region of the feminine pad of Figure 1A.
Figure 2A is an illustration of a first wing of the feminine pad of Figure 1A.
Figure 2B is an illustration of a second wing of the feminine pad of Figure 1A.
Figure 3 is an illustration of another form of first and second wings outside the subject-matter of the claims.
Figure 4 is an illustration of another form of first and second wings in accordance with the present disclosure.
Figure 5 is an illustration of a feminine pad comprising a visual signal in accordance with the present disclosure.
Figure 6 is an illustration of a feminine pad attached to a panty with wings wrapped around the panty.
Figure 7A is an illustration of an underside of a feminine pad.
Figure 7B is an illustration of a first wing of the feminine pad of Figure 7A.
Figure 7C is an illustration of a second wing of the feminine pad of Figure 7A.
Figure 8 is an illustration showing a close up view of the first wing of Figure 7B.
Figure 9A is an illustration of the first wing in Figure 7B further showing coverage areas and a grasping area.
Figure 9B is an illustration of the second wing of Figure7C further showing coverage areas and a grasping area.
Figure 9C is an illustration of wings wrapped around a panty and also showing coverage areas of the wings.
Figure 10 is a schematic plan view of the feminine pad of Figure 1A with portions cut-away for ease of view of some of the internal features of the feminine pad.
Figure 11 is a schematic plan view of a secondary topsheet suitable for use with the feminine pads of the present disclosure.
Figure 12 is a photograph showing a feminine pad.
Figure 13 is a photograph showing another feminine pad.

### DETAILED DESCRIPTION OF THE INVENTION

The feminine pad according to the present disclosure can provide easily discernable placement and orientation guides which reduce the likelihood of misapplication by the user. The feminine pad according to the present disclosure can also provide facilitated attachment guides which can reduce the likelihood of misapplication of the feminine pad, but also can reduce the likelihood of improper attachment of the feminine pad to a panty. The feminine pad of the present disclosure includes a pair of wings which are uniquely shaped which acts as an easily discernable placement / orientation guide for a user. Additionally, the wings comprise a visual signal which can reduce the likelihood of improper attachment of the feminine pad to the panty. Each of these features is discussed in additional detail herein.

Pads of the present invention comprise a first end, a second end opposing the first end, and an intermediate region joining the first end and the second end. Each of these areas can play a role in providing intuitive pad orientation cues which in turn can provide intuitive pad attachment cues. Each of the first end, second end, and intermediate regions are discussed in additional detail herein. And, while the description herein references "feminine pads" or "feminine hygiene pads," the features of the article described herein shall be equally applicable to panty liners and adult incontinence pads.

### Pad Orientation / Placement

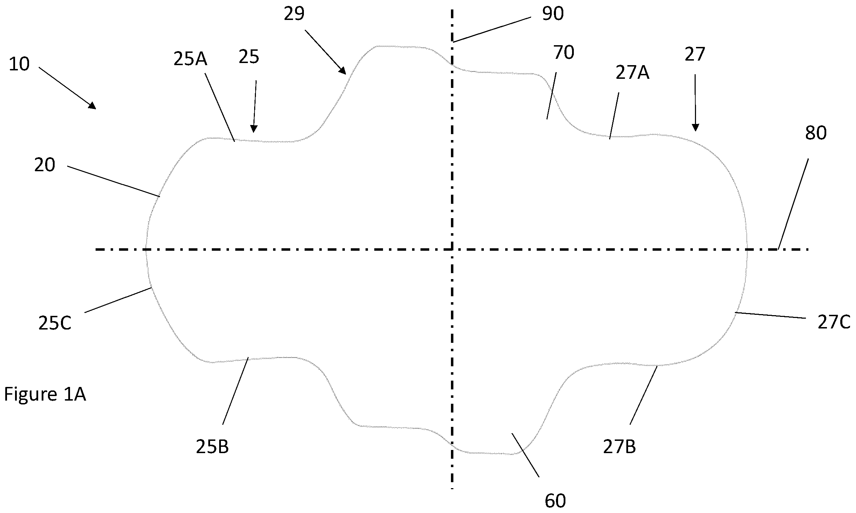

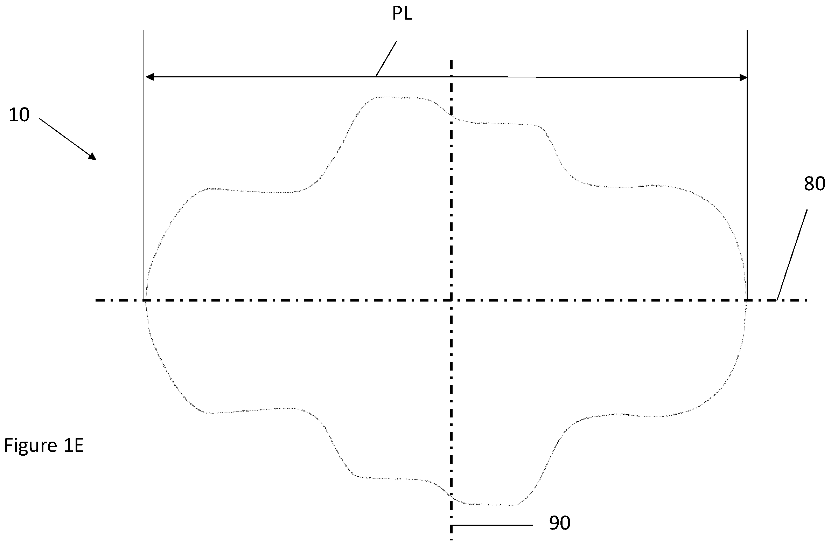

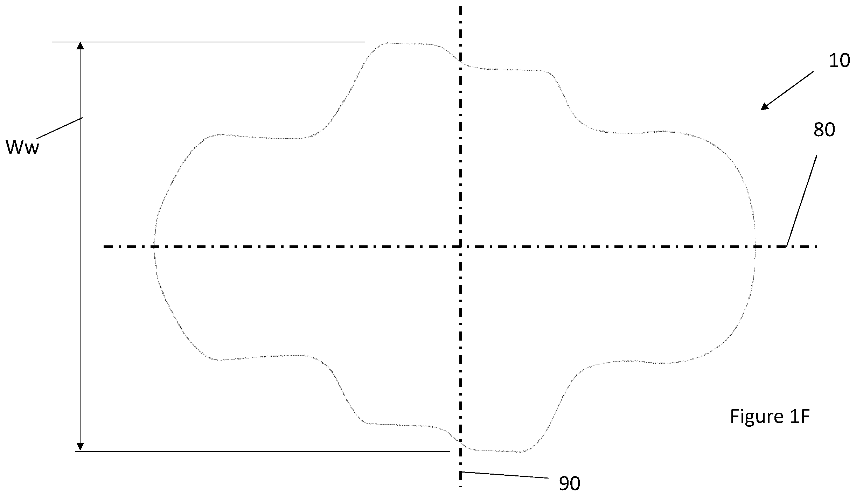

As mentioned previously, feminine hygiene pads of the present disclosure comprise a unique shape which facilitates orientation and placement of the feminine pad within a panty. A feminine hygiene pad constructed in accordance with the present disclosure is shown in Figure 1A. Figure 1A depicts an outer periphery of a feminine pad 10. The feminine pad 10 comprises a chassis 20 and a pair of wings 60 and 70 extending outboard of the chassis 20. The chassis 20 has a first end 25 and a second end 27 and an intermediate region 29 disposed between the first end 25 and the second end 27. The feminine pad 10 further comprises a longitudinal centerline 80 and a lateral centerline 90 which is disposed generally perpendicular to the longitudinal centerline 80. And, the chassis 20 further comprises a topsheet, a backsheet, and an absorbent core disposed between the topsheet and the backsheet. The topsheet, backsheet, and absorbent core are discussed in additional detail herein.

Regarding the chassis 20, the first end 25 comprises a pair of longitudinal side edges 25A and 25B and a first end edge 25C which joins the longitudinal side edges 25A and 25B. The longitudinal side edges 25A and 25B may be disposed generally parallel to the longitudinal centerline 80. Similarly, the second end 27 may comprise a pair of longitudinal side edges 27A and 27B which are disposed generally parallel to the longitudinal centerline 80. A second end edge 27C joins the longitudinal side edges 27A and 27B. Note that the term "generally parallel" does not require the longitudinal side edges to be absolutely parallel. In the specific case of the longitudinal side edges 25A, 25B, 27A and 27B, their respective inclination to the longitudinal centerline 80 is discussed in additional detail herein.

The delineation between the first end 25, second end 27, and intermediate region 29 can be dependent upon the extension of the wings from the chassis 20. For example, the delineation between the first end 25 and the intermediate region 29 can be approximated by determining a first intersection point between longitudinal side edge 25A and a leading edge of wing 70 and a second intersection point between longitudinal side edge 25B and a leading edge of wing 60. A first line generally parallel to the lateral centerline 90 extending from the first intersection point to the second intersection point, provides a useful approximation of the delineation between the first end 25 and the intermediate region 29. As another example, the delineation between the second end 27 and the intermediate region 29 can be determined in much the same way as described above. For example, finding a third intersection point between a trailing edge of wing 70 and the longitudinal side edge 27A and a fourth intersection point between a trailing edge of wing 60 and the longitudinal side edge 27B can be helpful. A second line generally parallel to the lateral centerline 90 extending from the third intersection point to the fourth intersection point, provides a useful approximation of the delineation between the second end 27 and the intermediate region 29. The pertinent edges of the wings 60 and 70 are discussed in additional detail hereafter.

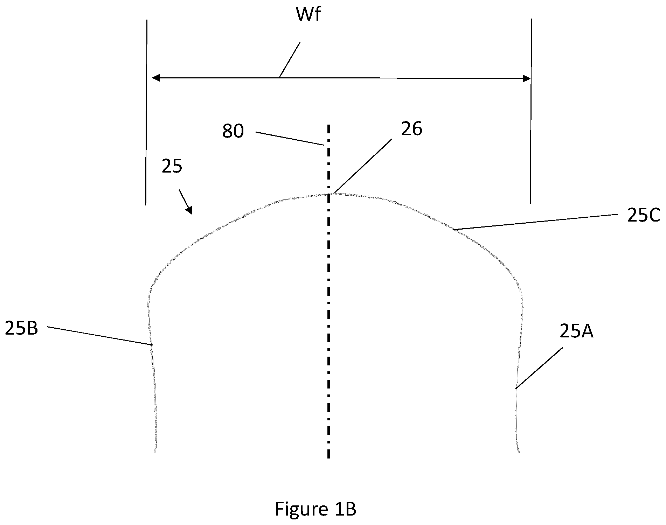

Focusing on the first end 25 in Figure 1B, it is worth noting that the first end edge 25C tapers toward an apex 26. The tapering of the first end edge 25C toward the apex 26 in the first end 25 can help a wearer identify the correct orientation of the feminine pad 10 in the panty. For example, due to the tapering of the first end edge 25C toward the apex 26, a user may be able to more easily identify a front of the feminine pad 10 as opposed to the second end edge 27C which is more rounded as discussed below.

As shown, the first end 25 may also comprise a width Wf which is generally parallel to the lateral centerline 90 (shown in Figure 1A). The width Wf represents the widest portion of the first end 25. The width Wf is discussed in additional detail hereafter.

Additionally, the first end edges 25A and 25B may be inclined slightly inward from the first end edge 25C. Such inclination by the first end edges 25A and 25B provide for a smaller width chassis 20 moving from the first end 25 toward the intermediate region 29. This inclination by the first end edges 25A and 25B may generally follow the contour of the panty thereby providing a more comfortable fit within the panty of the wearer.

Moreover, inclined first end edges 25A and 25B can increase the longevity of a rotary knife system. Rotary knife systems typically comprise a die cutting roll and an anvil roll and may be utilized to cut the final shape of the pad from their respective webs. Edges which are generally parallel to an MD direction (generally parallel to the longitudinal centerline 80) can prematurely wear the anvil roll. In such instances, blades of the die cutting roll strike the anvil roll in generally a small band width in a CD direction (generally parallel to the lateral centerline 90). Such repeated strikes can prematurely wear the die cutting roll and/or the anvil roll. However, where edges are inclined, such as first end edges 25A and 25B, blades of the die cutting roll strike the anvil roll along an increased band width in the CD direction. The larger band width on the anvil roll can extend the longevity of the anvil roll.

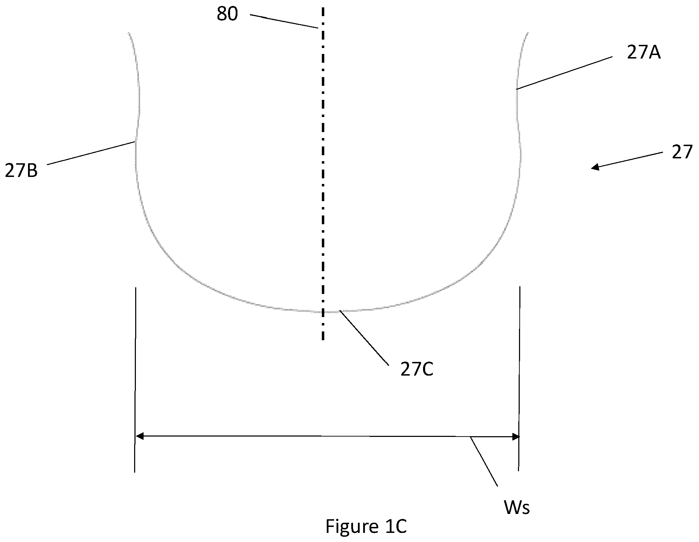

Focusing on the second end 27 in Figure 1C, the second end edge 27C, unlike the first end edge 25C (shown in Figure 1B), may be more rounded. Again, because of the difference between the first end edge 25C (shown in Figure 1B) and the second end edge 27C, the likelihood of misapplication of the feminine pad 10 to the panty may be decreased. For example, because of the shape differences between the first end edge 25C and the second end edge 27C, a user may be more inclined to orient the first end edge 25C in the front of the panty and the second end edge 27C in the back of the panty - which is the appropriate application.

Additionally, the second end 27 may comprise a width Ws which is generally parallel to the lateral centerline 90. The width Ws represents the widest portion of the second end 27 of the feminine pad 10. The width Ws is discussed in additional detail hereafter.

The side edges 27A and 27B of the second end 27 may be inclined inward from the second end edge 27C. Such inclination of the side edges 27A and 27B can provide for a smaller width chassis 20 moving from the second end 27 toward the intermediate region 29. Much like the first side edges 25A and 25B (shown in Figure 1B), the inclined second side edges 27A and 27B can provide longevity for the rotary knife system and can follow the contour of the panty to provide more comfort.

Referring now to Figures 1A-1C, as noted previously, the feminine pad 10 may comprise a first end width Wf and a second end width Ws. The width of the first end Wf may be from about 80 to 103 mm, more preferably from about 86 to 102 mm, or most preferably from about 90 mm to about 100 mm specifically including any values within these ranges and any ranges created thereby. The width of the second end Ws may be from about 80 mm to about 170 mm, more preferably from about 90 mm to about 140 mm, or most preferably from about 100 mm to about 120 mm specifically including any values within these ranges and any ranges created thereby. A ratio of Wf to Ws may be from about 0.47 to about 1.0, more preferably from about 0.75 to about 0.95, or most preferably from about 0.85 to about 0.90 specifically including any values within these ranges and any ranges created thereby. It is worth noting that Ws for longer pads may be wider to provide additional protection for the user. The second end on longer pads typically corresponds to additional panty area.

As noted, the width of the first end 25 may decrease toward the intermediate region 29. Similarly the width of the second end 27 may decrease toward the intermediate region 29. In some forms, the first end width may decrease to about 53 percent of Wf, more preferably 80 percent of Wf, most preferably about 95 percent of Wf specifically including any values within these ranges and any ranges created thereby. In some forms, independent of or inclusive of the decrease of the first end width, the second end width may decrease to 40 percent of Ws, more preferably 85 percent of Ws, most preferably about 98 percent of Ws specifically including any values within these ranges and any ranges created thereby.

Referring back to Figure 1A, the intermediate region can also contribute to the easily discernable indication of orientation and placement of the feminine pad 10. The intermediate region 29, corresponds to an area of the pad which comprises a target fluid entry zone. Additionally, the intermediate region 29 comprises wings 60 and 70 which extend outboard of the chassis 20. The wings of the present disclosure can help provide a user with a placement guide such that the vaginal opening corresponds to the intermediate region 29 and the target fluid entry zone of the feminine pad.

The target fluid entry zone of the feminine pad 10 should line up with the vaginal opening. And the target fluid entry zone for the feminine pads of the present disclosure can be identified by drawing a first imaginary line connecting the first intersection point between longitudinal side edge 25A and a leading edge of wing 70 and the fourth intersection point between a trailing edge of wing 60 and the longitudinal side edge 27B. A second imaginary line connecting the second intersection point between longitudinal side edge 25B and a leading edge of wing 60 and the third intersection point between a trailing edge of wing 70 and the longitudinal side edge 27A can also be drawn. An intersection between the first imaginary line and the second imaginary line can provide a useful approximation for the target fluid entry zone.

The wings of the present disclosure comprise various zone / areas which can facilitate orientation and/or placement of the feminine pad within a panty. Bisecting lines will be utilized hereafter to describe the various zones / areas of the wings of the feminine pads in accordance with the present disclosure. In some forms, one or more of the bisecting lines may be co-linear with the lateral centerline 90 of the feminine pad. In some forms, the bisecting lines may be offset from the lateral centerline. For example, one or more of the bisecting lines may be on a first side of the lateral centerline 90 - more proximal to the first end. As another example, one or more of the bisecting lines may be on a second side of the lateral centerline 80 - more proximal to the second end. As yet another example, one bisecting line may be on a first side of the lateral centerline while a second bisecting line may be on a second side of the lateral centerline. As yet another example, the bisecting lines may be co-linear with each other. And in some forms, the wings described herein are asymmetric about their respective bisecting line.

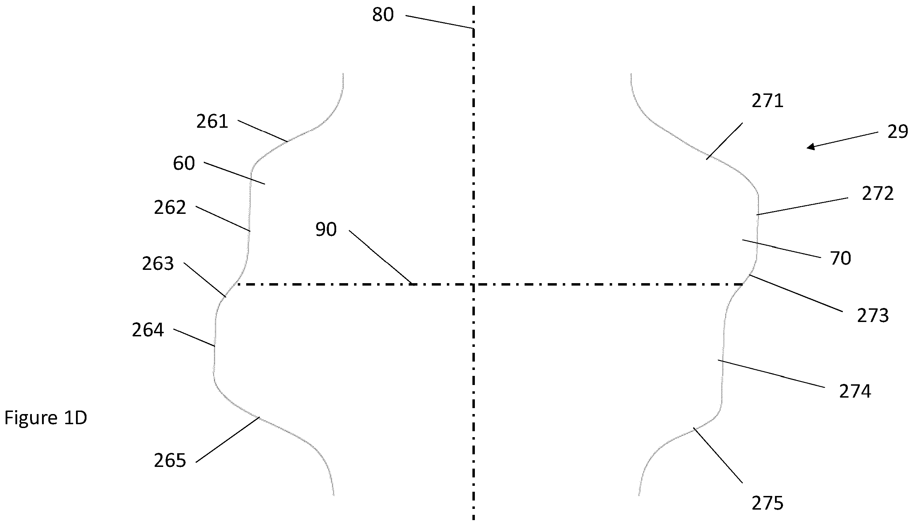

Focusing on the intermediate region 29 in Figure 1D, the wings 60 and 70 are discussed in additional detail. Each of the wings 60 and 70 comprise an outer periphery defined, in part, by their respective leading edges and trailing edges. Additional edges are disposed between the leading edges and trailing edges connecting the leading and trailing edges together.

As shown, wing 60 comprises a leading edge 261 and a trailing edge 265. The leading edge 261 is disposed more proximal to the first end 25 than the second end 27 (each shown in Figure 1A). Conversely, the trailing edge 265 is disposed more proximal to the second end 27 than the leading edge 261. Each of the leading edge 261 and the trailing edge 265 extend outboard of the chassis 20 and outboard of the longitudinal side edges 25B and 27B (each shown in Figure 1A).

In some forms outside the subject-matter of the claims, wing 60 may further comprise a first proximal edge 262 which extends from the first leading edge 261 away from the first end 25 and generally parallel to the longitudinal centerline 80. A first transition edge 263 extends from the first proximal edge 262 slightly outboard of the first proximal edge 262 and more proximal to the second end 27. A first distal edge 264 extends from the first trailing edge 265 toward the first end 25 and intersects with the first transition edge 263.

As shown and outside the subject-matter of the claims, the first proximal edge 261 and the first distal edge 264 are both disposed outboard of the sides edges 25B and 27B. However, the first distal edge 264 is disposed outboard by a greater extent than the first proximal edge 261. As noted previously, the wings of the present disclosure comprise various zones and areas. Such zones will be described hereafter.

Wing 70 may be configured similarly with regard to wing 60. For example, wing 70 comprises a second leading edge 271 and a second trailing edge 275. The leading edge 271 is disposed more proximal to the first end 25 than the second end 27. Conversely, the trailing edge 275 is disposed more proximal to the second end 27 than the leading edge 271. Each of the leading edge 271 and the trailing edge 275 extend outboard of the chassis 20 and outboard of the longitudinal side edges 25A and 25B (shown in Figure 1A).

Wing 70 further comprises a second distal edge 272 which extends from the second leading edge 271 away from the first end 25 and generally parallel to the longitudinal centerline 80. In forms outside the subject-matter of the claims, a second transition edge 273 extends from the second distal edge 272 slightly inboard of the second distal edge 272 and more proximal to the second end 27. A second proximal edge 274 extends from the second trailing edge 275 toward the first end 25 and intersects with the second transition edge 273.

As shown and outside the subject-matter of the claims, the second proximal edge 274 and the second distal edge 272 are both disposed outboard of the sides edges 25B and 27B. However, the second distal edge 272 is disposed outboard by a greater extent than the second proximal edge 274. As noted previously, the wings of the present disclosure comprise various zones and areas. Such zones will be described hereafter.

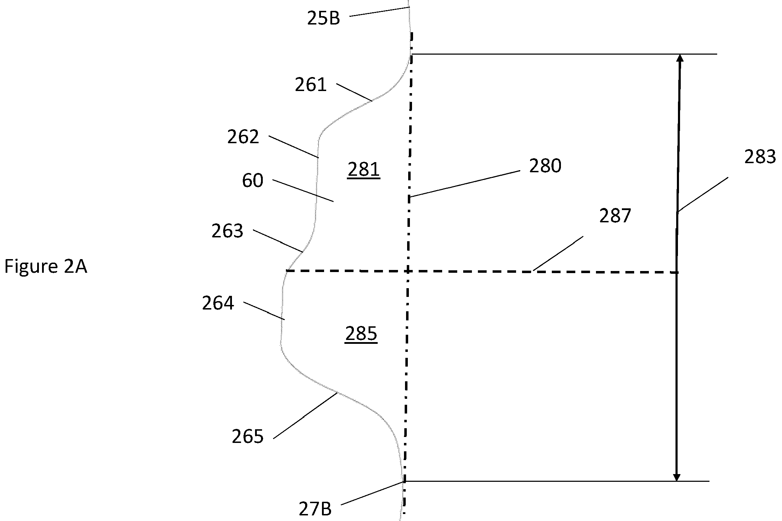

As shown in Figure 2A, the wing 60 may have a length 283 which is generally parallel to longitudinal centerline. The length 283 is defined by an intersection of the leading edge 261 and the side edge 25B and an intersection of the trailing edge 265 and the side edge 27B. A bisecting line 287 which bisects the length 283 is generally parallel to the lateral centerline 90 (shown in Figure 1A) and can serve as a boundary between a first proximal zone 281 and a first distal zone 285. As shown, the bisecting line 287 may intersect the first distal edge 264, the first transition edge 263 or the first proximal edge 262 depending on the design of the wing 60. Additionally, a fold line 280 may be created for the wing 60. The intersections which defines the length 283 similarly defines the endpoints of the fold line 280.

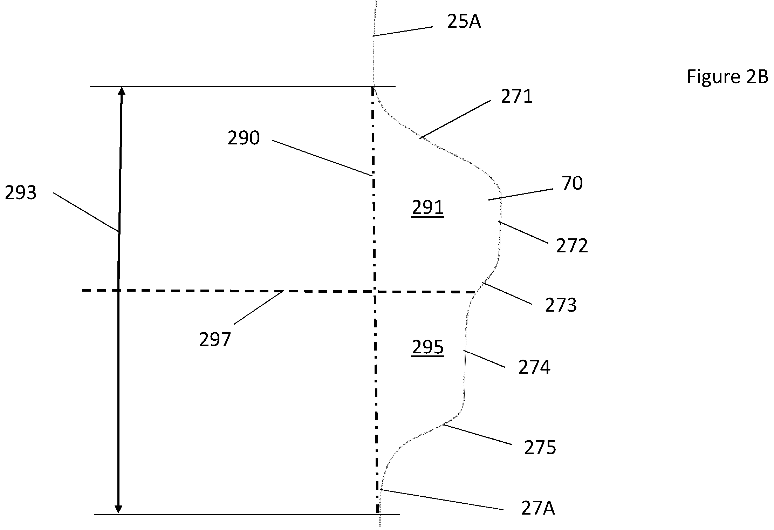

In some forms outside the subject-matter of the claims, for wing 60, the first proximal zone 281 is defined by the first leading edge 261, the first proximal edge 262, the first bisecting line 287, the fold line 280, and depending on the shape of wing 60, the first transition edge 263. In some forms outside the subject-matter of the claims, the first bisecting line 287 may intersect the first proximal edge 262. In such forms, the first transition edge 263 would not form part of the boundary of the first proximal zone 281. In other forms, the first bisecting line 287 may intersect the first distal edge 264. In such forms, the first distal edge 264 would form part of the boundary of the first proximal zone 281. The first distal zone 285 is defined by the first bisecting line 287, the fold line 283, the first trailing edge 265, the first distal edge 264, and depending on the shape of the wing, the first transition edge 263. As shown, the first distal zone 285 may comprise the laterally outermost extents of the wing 60
Wing 70 may be similarly constructed as discussed with regard to wing 60. As shown in Figure 2B, the wing 70 may have a length 293 which is generally parallel to longitudinal centerline. The length 293 is defined by an intersections of the leading edge 271 and the side edge 25A and an intersection of the trailing edge 275 and the side edge 27A. A second bisecting line 297 which bisects the length 293 is generally parallel to the lateral centerline 90 (shown in Figure 1A) and can serve as a boundary between a second proximal zone 295 and a second distal zone 291. As shown but outside the subject-matter of the claims, the second bisecting line 297 may intersect the second transition edge 273, the second distal edge 272 or the second proximal edge 274. Additionally, a fold line 290 may be created for the wing 70. The intersections which define the length 293 similarly define the endpoints of the fold line 290.

In some forms, for wing 70, the second distal zone 291 is defined by the second leading edge 271, the second distal edge 272, the second bisecting line 297, the fold line 290, and depending on the shape of wing 70, the second transition edge 273. In some forms, the second bisecting line 297 may intersect the second distal edge 272. In such forms, the second transition edge 273 would not form part of the boundary of the second distal zone 291. As shown in Figure 2B, the second distal zone 291 may comprise the laterally outermost extents of wing 70. In some forms, the second bisecting line 297 may intersect the second proximal edge 274. In such forms, the second proximal edge 274 may form at least a portion of the boundary of the second distal zone 291. The second proximal zone 295 is defined by the second bisecting line 297, the fold line 290, the second trailing edge 275, the second proximal edge 274 and, depending on the shape of the wing, the second transition edge 273.

Referring now to Figures 2A and 2B, the length 283 of wing 60 and the length 293 of wing 70 may be equal or at least within about 10 percent of one another. However, forms of the present invention are contemplated where the lengths 283 and 293 of the wings 60 and 70, respectively differ by more than about 20 percent, more than about 30 percent, or more than about 50 percent.

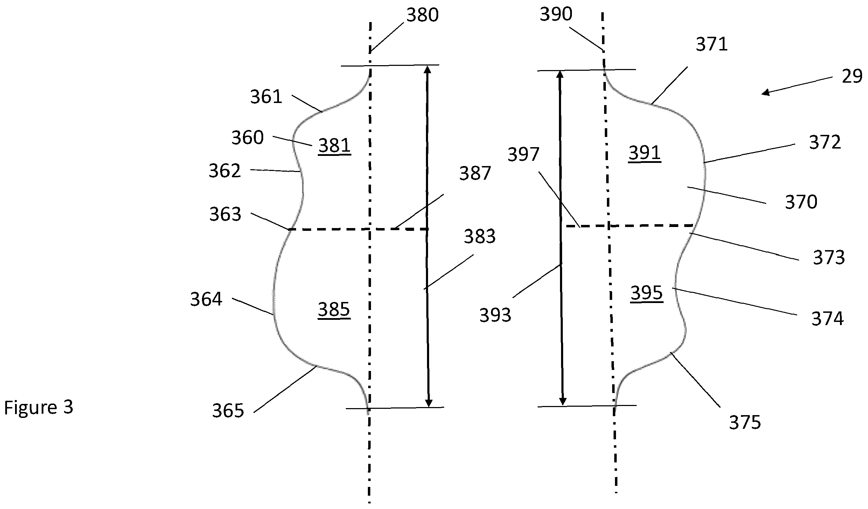

Figure 3 depicts another version of the intermediate region 29 of the feminine pad 10 outside the subject-matter of the claims. Similar to wing 60 discussed above, wing 360 may comprise a first leading edge 361 and a first trailing edge 365. A first proximal edge 362 may extend from the first leading edge 361 toward the first trailing edge 365. However, unlike the first proximal edge 262 (shown in Figures 1D and 2A), the first proximal edge 362 may be more curvilinear in nature. A first transition edge 363 may also be much more curvilinear than its counterpart 263 (shown in Figures 1D and 2A). A first distal edge 364 extends from the first transition edge 363 and intersects the first trailing edge 365. The zones of wing 360 may be determined similar to the methodology described above.

Wing 370 may comprise a second leading edge 371 and a second trailing edge 375. A second distal edge 372 may extend from the second leading edge 371 toward the second trailing edge 375. However, unlike the second distal edge 272 (shown in Figures 1D and 2B), the second proximal edge 372 may be more curvilinear in nature. A second transition edge 373 may also be much more curvilinear than its counterpart 273 (shown in Figures 1D and 2B). A second proximal edge 374 extends from the second transition edge 373 and intersects the second trailing edge 375. The zones of wing 370 may be determined similar to the methodology described above.

For example, a length 383 of wing 360 may be determined by the intersection of the first leading edge 361 and the side edges of the chassis and the intersection of the first trailing edge 365 and the side edges of the chassis. The length 383 is generally parallel to the longitudinal centerline 80 (shown in Figure 1A). A first bisecting line 387 which bisects the length 383 may extend generally parallel to the lateral centerline 90 (shown in Figure 1A) and may intersect the first transition edge 363. **In** some forms outside the subject-matter of the claims, the first bisecting line 387 may intersect the first proximal edge 362 or the first distal edge 364, depending on the design of the wing 360.

As shown but outside the subject-matter of the claims, a first proximal zone 381 of wing 360 may be bounded by the first leading edge 361, the first proximal edge 362, the first bisecting line 387, the first fold line 380, and depending on the shape of the wing 360 the first transition edge 363. **In** some forms, the bisecting line 387 may intersect the first distal edge 364. **In** such forms, the first distal edge 364 may form a portion of the first proximal zone 381. **In** other forms, the bisecting line 387 may intersect the first proximal edge 362, in such forms, the first transition edge 363 may not form a portion of the first proximal zone 381.

A first distal zone 385 may be defined by the first trailing edge 365, the first distal edge 364, the first bisecting line 387, the fold line 383, and depending on the shape of the wing, the first transition edge 363. The first distal zone 385 may comprise the laterally outermost extents of the wing 360.

A wing 370 may be configured similarly to the wing 360. Namely, the wing 370 may comprise a length 393 which is determined by the intersection of the second leading edge 371 and the side edges of the chassis and the intersection of the second trailing edge 375 and the side edges of the chassis. A second bisecting line 397 extends from the fold line 393 generally parallel to the lateral centerline 90 (shown in Figure 1A) and intersects the second distal edge 372. In some forms but outside the subject-matter of the claims, the second bisecting line 397 may intersect the second proximal edge 374 or the second transition edge 363, depending on the design of the wing 370.

A second distal zone 391 and a second proximal zone 395 may be defined, in part, by the second bisecting line 397. The second distal zone 391 may comprise the laterally outermost extents of the wing 370. The second distal zone 391 may be defined by the second leading edge 371, the second distal edge 372, the second bisecting line 397, the fold line 393, and depending on the shape of the wing 370, the second transition edge 373. In some forms, the second bisecting line 397 may intersect the first distal edge 372. In such forms, the second transition edge 373 would not form part of the boundary of the second distal zone 391. In other forms but outside the subject-matter of the claims, the second bisecting line 397 may intersect the second proximal edge 374. In such forms, the second proximal edge 374 may form a portion of the boundary of the second distal zone 391.

A second proximal zone 395 may similarly be created by the second bisecting line 397. The second proximal zone 395 may be defined by the second trailing edge 375, the second proximal edge 374, the second bisecting line 397, the fold line 393, and depending on the shaped of the wing, the second transition edge 373.

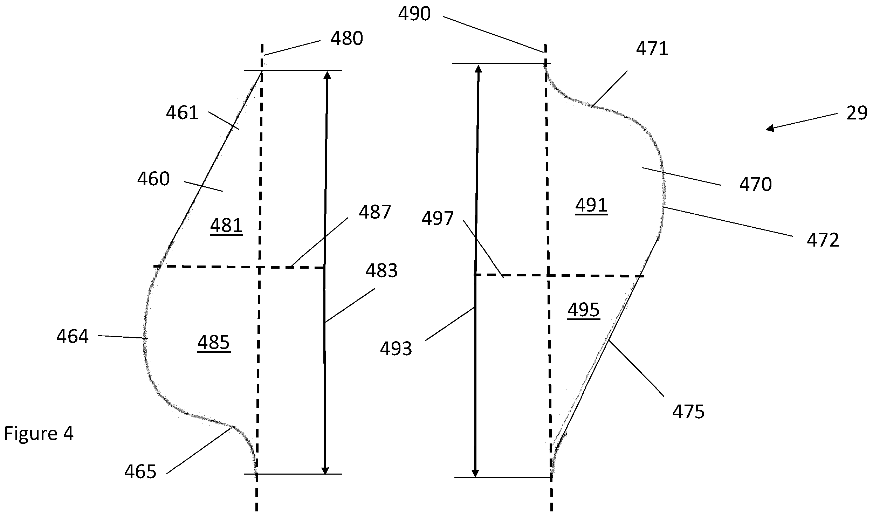

Figure 4 depicts yet another version of the intermediate region 29 of the feminine pad 10 of the present disclosure. As shown wing 460 comprises a leading edge 461 which extends from side edges of the chassis toward the second end of the feminine pad. As shown, the leading edge 461 extends toward the second end of the feminine pad and also extends outboard of the chassis. However, in the form shown, the wing 460 may not comprise a first proximal edge like the forms discussed heretofore. Instead, the first leading edge 461 transitions into a first distal edge 464. And, the first distal edge 464 transitions into a first trailing edge 465 which joins the first distal edge 464 and the chassis.

A length 483 of wing 460 may be determined by the intersection of the first leading edge 461 and the side edges of the chassis and the intersection of the first trailing edge 465 and the side edges of the chassis. The length 483 is generally parallel to the longitudinal centerline 80 (shown in Figure 1A). A first bisecting line 487 which bisects the length 483 may extend generally parallel to the lateral centerline 90 (shown in Figure 1A) and may intersect the first leading edge 461 or the first distal edge 464, depending on the design of the wing 460.

As shown, a first proximal zone 481 of wing 460 may be bounded by the first leading edge 461, the first bisecting line 487, and the first fold line 480. In some forms, the bisecting line 487 may intersect the first distal edge 464. In such forms, the first distal edge 464 may form a portion of the first proximal zone 481. A first distal zone 485 may be defined by the first trailing edge 465, the first distal edge 464, the first bisecting line 487, and the fold line 480. The first distal zone 485 may comprise the laterally outermost extents of the wing 460.

Wing 470 comprises a second leading edge 471 and a second trailing edge 475. A second distal edge 472 extends from the second leading edge 471 to the second trailing edge 475. A length 493 of wing 470 may be determined by the intersection of the second leading edge 471 and the side edges of the chassis and the intersection of the second trailing edge 475 and the side edges of the chassis. The length 493 is generally parallel to the longitudinal centerline 80 (shown in Figure 1A). A second bisecting line 497 which bisects the length 493 may extend generally parallel to the lateral centerline 90 (shown in Figure 1A) and may intersect the second trailing edge 475 or the second distal edge 472, depending on the design of the wing 470.

A second distal zone 491 and a second proximal zone 495 may be defined, in part, by the second bisecting line 497. The second distal zone 491 may comprise the laterally outermost extents of the wing 470. The second distal zone 491 may be defined by the second leading edge 471, the second distal edge 472, the second bisecting line 497, and the fold line 490. In some forms, the second bisecting line 497 may intersect the second distal edge 472. The second proximal zone 495 may similarly be created by the second bisecting line 497. The second proximal zone 495 may be defined by the second trailing edge 475, the second bisecting line 497, the fold line 490, and depending on the shape of the wing, the second distal edge 472.

As shown in Figures 1A-4, the wings are asymmetric with respect to each other. Such asymmetry can contribute to the overall indication of the correct placement and correct orientation of the feminine pad within the panty. And, as noted previously, the correct placement and correct orientation can allow the absorbent core and other portions of the feminine pad to function with more efficacy than if improperly placed or oriented.

For each of the wings shown in Figures 2A-4, each is asymmetric about its bisecting line. Again, this asymmetry can help facilitate application of the pad to the panty. And, such asymmetry can similarly facilitate removal of the pad from the panty as described herein. As shown, each of the distal zones has a larger surface area than their respective proximal zones.

### Proper attachment of the pad

In order to facilitate fastening the wings onto the panty of a wearer, the wearer should be appropriately apprised regarding where to grasp the wings. Improper handling of the wings could produce wrinkles in the wings during fastening. These wrinkles can cause discomfort and may lead to premature detachment of the feminine pad from the panty. For example, referring back to Figures 2A and 2B, if the user were to grasp either the wing 60 or 70 in the first proximal zone 281 or the second proximal zone 295, respectively, wrinkles could be created in each of the wings 60 and 70. The inventors have found that the better approach to reduce the likelihood of wrinkles within the wings during fastening occurs when the wings are grasped by their respective distal zones. Because the distal zones extend outboard to a further extent than does the proximal zones, the distal zones can more easily manipulate the proximal zones. While grasping each of the wings at two different longitudinal locations may seem counterintuitive, such application can enhance the experience provided to the wearer during use. For example, as noted previously, such application can reduce the likelihood of wrinkles in the wings which can enhance the comfort to the user.

In order to advocate proper application of the wings to the panty, visual signals are employed which help provide an indication of where to grasp the wings. For example, referring to Figures 2A and 2B, in some forms, embossing may be provided in the first distal zone 285 of wing 60 and the first distal zone 291 in wing 70. As another example, a visual signal may comprise printing in the first distal zone 285 of wing 60 and in the second distal zone 291 of wing 70. In yet another example, a visual signal may comprise a combination of printing and embossing in the first distal zone 285 and the second distal zone 291.

To avoid confusion during the application of the wings 60 and 70, the density of the visual signals on the wings 60 and 70 should be higher in the first distal zone 285 and second distal zone 291 than in the first proximal zone 281 and the second proximal zone 295, respectively. For example, where the visual signal comprises embossing, the level of embossing in the first distal zone 285 should be higher than the level of embossing in the first proximal zone 281. In such examples, the amount of surface area of the respective zones may be compared to determine which zone has a higher level of embossing. Where the visual signal comprises printing, the amount of surface area of the respective zones may be compared to determine which zone has a higher level of printing thereon. Where the visual signal comprises a combination of embossing and printing, the amount of surface area of the respective zones may be compared to determine which zone has a higher level of embossing or printing.

In some specific forms, the visual signal may be more direct and may not subscribe to the above. For example, in some forms, the visual signal may comprise an arrow or something similar which connotes a direction which is disposed adjacent to the intersection between the first trailing edge 265 and the first distal edge 264 or the intersection between the second leading edge 271 and the second distal edge 272. In such forms, the visual signal may need to be placed within 10 mm preferably within 8 mm, more preferably within 6 mm, most preferably within 5 mm of the first distal edge 264 of the wing 60 and/or the second distal edge 272 of the wing 70 specifically including any values within these ranges and any ranges created thereby.

While alternative wing configurations are contemplated, without implementation of the above visual signal, the application of the wings could lead to wrinkles and or other asperities in application of the feminine pad. And as stated before, such wrinkles may lead to premature detachment of the pad from the panty or may preclude attachment to the panty altogether. Alternative wing configurations are provided with regard to Figures 3 and 4.

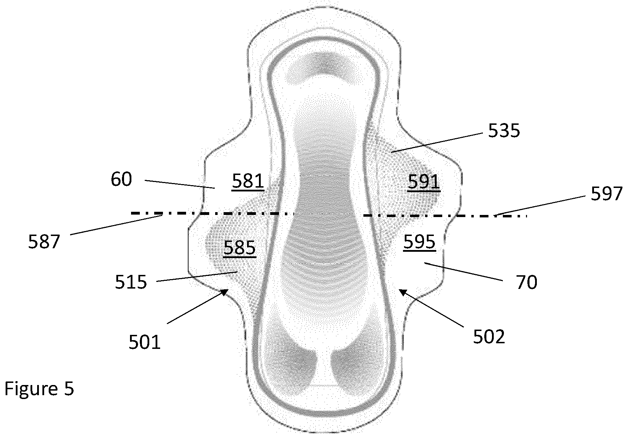

An example of a visual signal provided on the wings of the feminine pads of the present disclosure is provided with regard to Figure 5. As previously discussed, the distal zones comprise a higher amount of visual signaling than their proximal zone counterparts. Such signaling can facilitate the application of the feminine pad 10 (shown in Figure 1A) by prompting a user to grasp the wings 60, 70, 360, 370, 460, and 470 (shown in Figures 1A-4), in the appropriate locations for fastening about the panty of the wearer. Methods for measuring the amount of visual signaling are disclosed herein. Note that bisecting lines 587 and 597 have been provided which approximates a boundary between distal and proximal zones for the wings 60 and 70, respectively.

As shown, a print signal 515 may be disposed on a first surface 501 of wing 60 primarily in a distal zone 585 of the wing 60. While the print signal 515 may also exist in a proximal zone 581 of wing 60, a larger area of print is provided in the distal zone 585 of the wing 60.

Wing 70 may be similarly configured. For example, a print signal 535 may be disposed, primarily, on a first surface 502 in a distal zone 591 of the wing 70. While the print signal 535 may also exist in a proximal zone 595 of wing 70, a larger area of print is provided in the distal zone 595 of the wing 70.

And as stated previously, the visual signals described herein are not limited to print signals. In some forms, embossing may be utilized to provide the indication described herein. In other forms, a combination of print and embossing may be utilized. In other forms, different colored materials, e.g. dyed materials, may be utilized to signal to the user where to grasp.

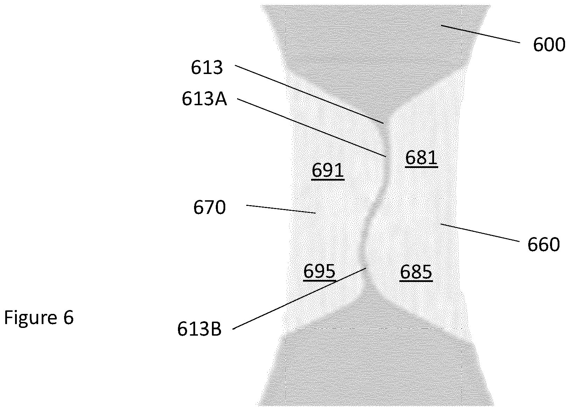

The wings, in a fastened configuration, can provide another visual attribute indicating proper attachment to the panty. Figure 6 is a depiction of a pair of wings 660 and 670 in a fastened configuration wrapped around a panty 600. As shown, wings 660 and 670 appear to nest when fastened to the panty. Specifically, a second distal zone 691 nests with a first proximal zone 681. And similarly, a first distal zone 685 nests with a second proximal zone 695.

The wings 670 and 660 may be designed such that for normal panties (excludes tanga underwear) the wings 660 and 670 do not overlap in use. As such, a gap 613 may exist between the wings 660 and 670 during use. A gap 613 may be highly variable as users can apply the wings of the present disclosure in a variety of different configurations and as panty crotch widths can vary greatly. For example, some users may attach the pad of the present disclosure to their panty such that the wings have a 0 mm gap between the wings. As another example, some users may fasten the wings to the panty such that the wings are broadly spaced apart, e.g. 3 mm, 5 mm, 7 mm, or 10 mm.

Additionally, the gap 613 can provide a visual indication of when the feminine pad is skewed with respect to the panty 600. For example, where the second distal zone 691 overlaps or contacts the first distal zone 685, this can indicate that the pad is skewed with respect to the panty 600. As another example, where the gap 613 has an uneven width from a first offset portion 613A to a second offset portion 613B, this can indicate skew with regard to the pad with respect to the panty 600. And, while a user may not be able to visualize the orientation of the wings 660 and 670 on the underside of the panty 600 during application, the gap 613 can provide a tactile cue to the user as to whether the pad is skewed with respect to the panty 600.

Additionally, the gap 613 may facilitate removal of the feminine pad. Where wings overlap, wing adhesive can adhere one wing to another of the wings. This can increase the difficulty of separating the wings from the panty during removal. Generally, in this scenario, a user would have to separate the wings from one another prior to removal of the wings from the panty.

As shown, the gap 613 may comprise offset portions 613A and 613B. As shown, portion 613A may be offset from the longitudinal centerline 80 (shown in Figure 1A) in a first direction, and portion 613B may be offset from the longitudinal centerline 80 (shown in Figure 1A) in a second direction which is opposite the first direction. In some forms, the first portion 613A may be aligned with the longitudinal centerline 80 (shown in Figure 1A) while the second portion 613B may be offset from the longitudinal centerline 80 (shown in Figure 1A). In other forms, the first portion 613A may be offset with respect to the longitudinal centerline 80 (shown in Figure 1A) while the second portion 613B is aligned with the longitudinal centerline 80 (shown in Figure 1A).

### Sustaining Coverage

In addition to providing assistance with the proper orientation of the pad, the shape of the wing can also play a large part in sustaining coverage and maintaining attachment to the panty. For example, wings of the present disclosure may be configured such that an adequate amount of fastening adhesive is provided to the wings so that the wings can maintain adherence to the panty. At the same time, a careful balance should be achieved. For example, too much adhesive can make the removal of the pad from the panty a much more arduous task. In contrast too little adhesive or ill placed adhesive can cause premature detachment of the pad from the panty.

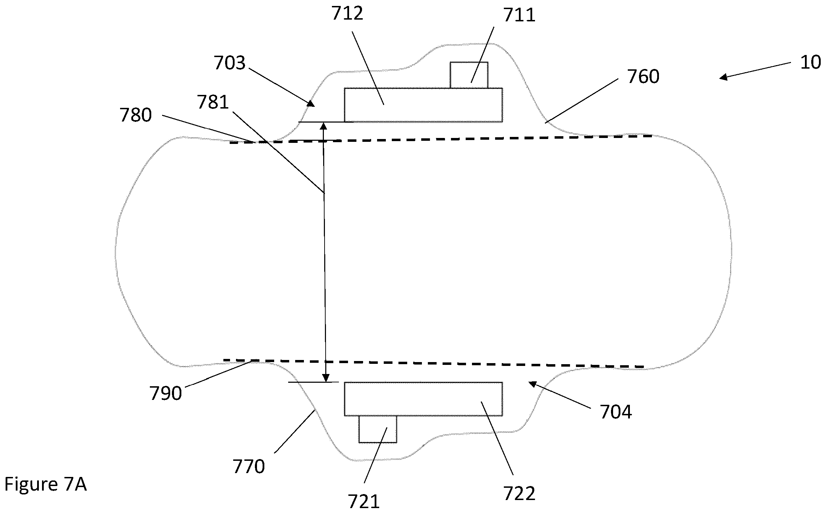

Referring to Figure 7A, in addition to the visual signaling discussed herein, the dimensions of the distal zones can be an important aspect of the feminine pads of the present invention as well as the adhesive placement on the wings. The underneath side of the feminine pad 10 is depicted with adhesive areas 711 and 712 on a second surface 703 of wing 760 and adhesive areas 721 and 722 on a second surface 704 of wing 770.

Focusing on wing 760, the adhesive areas 711 and 712 help secure the wing 760 to the panty in use. However, if the adhesive areas 711 and 712 are misapplied to the wing 760, then the wing 760 may become more susceptible to detachment during wear. So, the adhesive areas 711 and 712 should be carefully placed upon the wing 760.

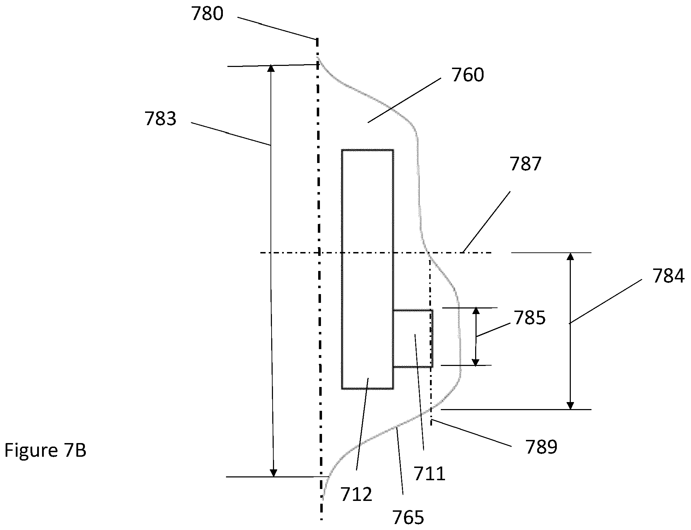

As shown in Figure 7B, the wing 760 comprises a length 783 which can be determined as described heretofore with regard to lengths associated with wings 60 and 70 (shown in Figures 2A and 2B, respectively). A bisecting line 787 - generally parallel to the lateral centerline 90 (shown in Figure 1A) bisects the length 783. A first guide line 789 is drawn from an intersection between the bisecting line 787 and an edge of the wing 760, e.g. first leading edge, first transition edge, or the first distal edge. The first guide line 789 extends perpendicular to the bisecting line 787 and extends parallel to the longitudinal centerline 90 (shown in Figure 1A). The first guide line 789 should extend into the distal zone or through the distal zone if its origin is already there such that the majority of the first guide line 789 resides in the distal zone of the wing 760. For wing 760, this may mean that the first guide line 789 will extend in a direction which is toward the second end of the feminine pad.

A distal zone adhesive length 784 is defined by the intersection between the bisecting line 787 and the first guide line 789 and an intersection between the first guide line 789 and a trailing edge 765 of wing 760. An adhesive length 785 is generally parallel to the longitudinal centerline 90 (shown in Figure 1A). The adhesive length 785 is determined by the outermost extents of the adhesive area 711 that are disposed on the wing 760.

A ratio of the distal zone adhesive length 784 to the adhesive length 785 can be important in ensuring that the wings do not prematurely detach and in ensuring that the wings are easy to attach and remove. For example, if the ratio is too high, then much of the distal zone of wing 760 remains without adhesive and can become prematurely detached during use. In contrast, if the ratio is too small, the user may not have a sufficient area to grasp the wing 760 during removal. Additionally, if the ratio is too small, the adhesive areas 711 and 712 may extend beyond the edges of the wings frustrating the application and removal of the pad from the panty.

Where the ratio is small, pad converting processes may need to be adjusted to ensure that the likelihood of adhesive areas 711 or 721 extending beyond the edges of the wings is minimized. For example, during processing, webs often track (move in a cross direction and machine direction). Web tracking can impact the placement of the adhesive areas on the wings. As another example, adhesive may be applied to the wings via a carrier sheet. There is variability of the adhesive application to the carrier sheet which can in turn impact the location of the adhesive areas on the wings. And, prior to packing the feminine pad, the wings are typically folded over a topsheet of the feminine pad. There is variability in the folding operation which can impact the placement of the adhesive on the wings.

Based on the foregoing, a balance should be achieved regarding the ratio of the distal zone adhesive length 784 to the adhesive length 785 to ensure that adequate adhesive coverage is achieved along with facilitated removal when desired. In some forms the ratio of the distal zone adhesive length 784 to the adhesive length 785 may be between about 1.15 to about 4.6, more preferably about 1.27 to about 2.25, or most preferably about 1.38 to about 2.0, specifically including any values within these ranges and any ranges created thereby.

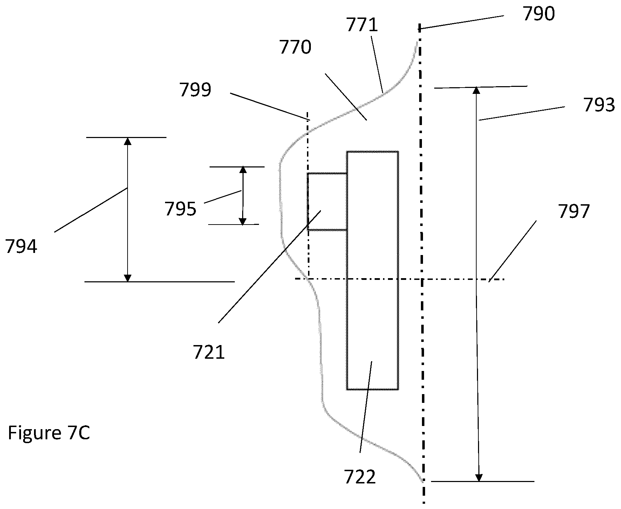

As shown in Figure 7C, the wing 770 comprises a length 793 which can be determined as described heretofore with regard to lengths associated with wings 60 and 70 (shown in Figures 2A and 2B, respectively). A bisecting line 797 - generally parallel to the lateral centerline 90 (shown in Figure 1A) bisects the length 793. A second guide line 799 is drawn from an intersection between the bisecting line 797 and an edge of the wing 770, e.g. second leading edge, second transition edge, or the second distal edge. The second guide line 799 extends perpendicular to the bisecting line 797 and extends parallel to the longitudinal centerline 90 (shown in Figure 1A). The second guide line 799 should extend into the distal zone or through the distal zone if its origin is already there such that the majority of the first guide line 799 resides in the distal zone of the wing 770. For wing 770, this may mean that the second guide line 799 will extend in a direction which is toward the first end of the feminine pad.

A distal zone adhesive length 794 is defined by the intersection between the bisecting line 797 and the second guide line 799 and an intersection between the second guide line 799 and a leading edge 771 of wing 760. An adhesive length 795 is generally parallel to the longitudinal centerline 90 (shown in Figure 1A). The adhesive length 795 is determined by the outermost extents of the adhesive area 721 that are disposed on the wing 770. A ratio of the distal zone adhesive length 794 to the adhesive length 795 can be within the ranges described heretofore with regard to wing 760.

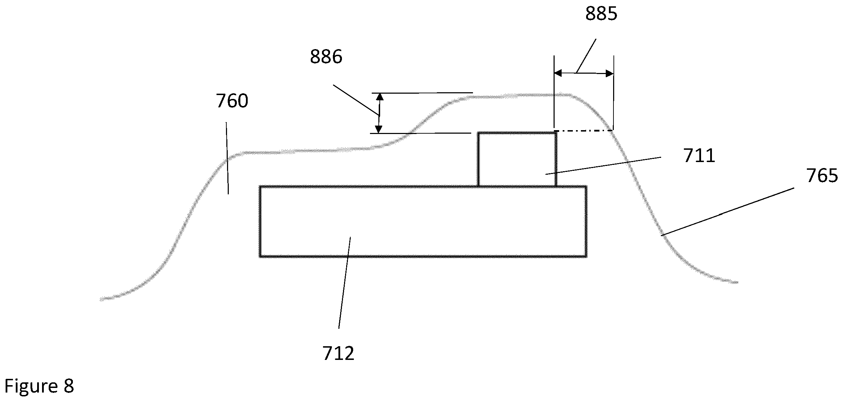

Referring to Figures 7B-8, spacing of the adhesive area 711 from the trailing edge 765 (or the leading edge if wing 770) may be of import in addition to the ratios discussed above. For, example, as noted previously, where the adhesive area 711 extends to the edges of the wing 760 or 770, the wing may prove difficult to remove from the panty. Instead, the inventors have found that a small handle (an area free of adhesive) can facilitate removal. A leading edge / trailing edge spacing 885 (generally parallel to the longitudinal centerline 80 (shown in Figure 1A)) can be about 2 mm to about 9 mm, more preferably about 3 mm to about 7 mm, or most preferably from about 4 mm to about 6 mm specifically including any values within these ranges and any ranges created thereby.

Similar spacing considerations may be taken into account regarding a distal edge spacing 886. The distal edge spacing 886 is generally parallel to the lateral centerline 80 (shown in Figure 1A). Similar to the leading edge / trailing edge spacing 885, the distal edge spacing 886 may be about 2 mm to about 9 mm, more preferably about 3 mm to about 7 mm, or most preferably from about 4 mm to about 6 mm specifically including any values within these ranges and any ranges created thereby.

Referring now to Figures 5 and 7A, as noted previously, the first print signal 515 and the second print signal 535 can provide the user with the appropriate indication about where to grasp the wings for proper attachment to the panty. Additionally, though, the first print signal 515 and the second print signal 535 can provide the user with appropriate indication of where the adhesive areas are on the second surface of the wing. For example, the first print signal 515 may correspond to the first adhesive area 711 on second surface 703 of the first wing 760 such that at least a portion of the first adhesive area 711 is disposed within the projection of the first print signal 515 area through the first wing 760. In some forms, at least 50 percent of the first adhesive area 711 is disposed within the projected area of the first print signal 515, more preferably at least 75 percent of the first adhesive area 711, or most preferably at least 100 percent of the first adhesive area 711, specifically including all values within these ranges and any ranges created thereby.

Referring back to Figure 7A, another factor of import is the spacing of the adhesive areas 712 and 722 from folding lines 780 and 790 for wings 760 and 770, respectively. The adhesive area 712 should be spaced adequately from the folding line 780 to ensure that the adhesive area 712 does not overlap or lie on the panty leg elastics in the fastened position. Similarly, the adhesive area 722 should be adequately spaced from the folding line 790 to ensure that the adhesive area 722 does not overlap or lie on the panty leg elastics in the fastened position. To accommodate this aspect, the adhesive areas 712 and 722 may be spaced apart from one another by a distance 781. In some forms, the adhesive areas 712 and 722 may have a space between them having a width 781 of from 70 mm to 120 mm, more preferably 80 mm to 110 mm, or most preferably 97.5 mm to about 102 mm, specifically including any values within these ranges and any ranges created thereby.

Without such spacing of the adhesive areas 712 and 722, the leg elastics of the panty could be rendered basically useless as the adhesive effectively locks the elastics in place. This can cause discomfort. Additionally, because the leg elastics are designed to flex and move with the wearer, during use, the leg elastics may prematurely cause the adhesive areas 712 and 722 to become detached from the panty. This detachment can further lead to premature detachment of the adhesive areas 711 and 721.

Another variable of import regarding sustained attachment to the panty is with regard to a grasping area versus coverage areas of the wings. In general, a ratio of coverage areas should be about equal. If the ratio of areas becomes unbalanced, then premature detachment of the pad from the panty can occur.

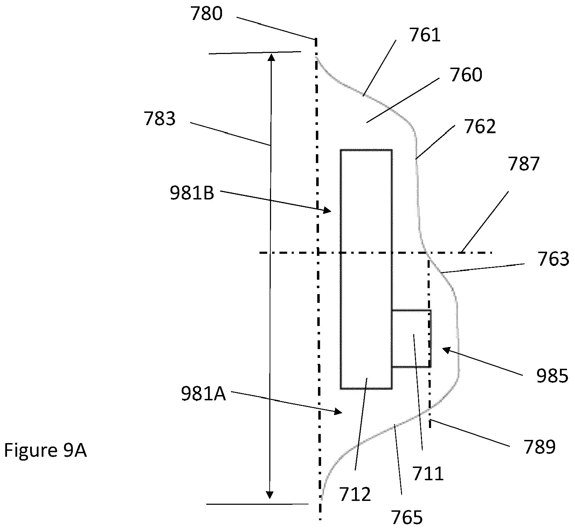

Referring to Figure 9A, the first guide line 789 associated with adhesive area 711 is helpful in determining a grasping area 985 versus the coverage areas 981A and 981B. The first guide line 789 can create a boundary for the grasping area 985, a first coverage area 981A, and a second coverage areas 981B on the wing 760. As shown, the first coverage area 981A is bounded by the first trailing edge 765, the first guide line 789, the bisecting line 787, and the fold line 780. The second coverage area 981B is bounded by a first leading edge 761, a first proximal edge 762, the bisecting line 787 and the fold line 780. In some forms, the first coverage area 981A and the second coverage area 981B are about the same size.

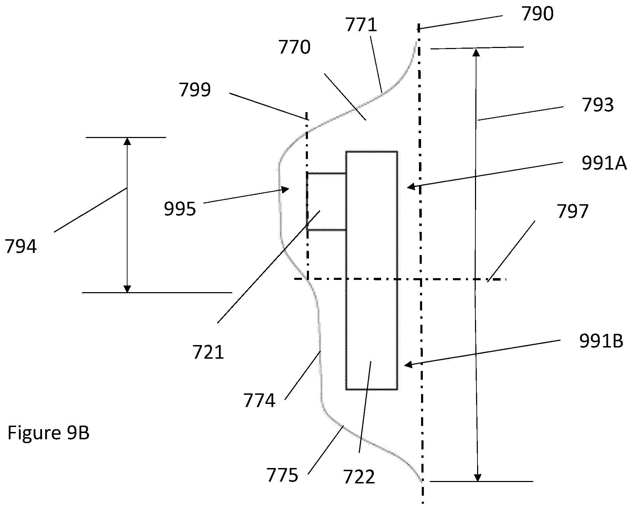

Referring to Figure 9B, the second guideline 799 is helpful in determining a grasping area 995 and coverage areas 991A and 991B for the wing 770. The second guide line 799 can create a boundary for the grasping area 995, the first coverage area 991A and the second coverage area 991B on the wing 770. As shown, the first coverage area 991A is bounded by the second leading edge 771, the second guide line 799, the bisecting line 797, and fold line 790. Similarly, the second coverage area 991B is bounded by a second trailing edge 775, a second proximal edge 774, the bisecting line 797, and fold line 790. In some forms, the first coverage area 991A and the second coverage area 991B are about the same size.

It is worth noting that the first guide line 789 and the second guide line 799, in some forms, can form a portion of a boundary for the second coverage areas 981B and 991B. Depending on the placement of the adhesive areas 711 and 721, the first guide line 789 and/or second guide line 799 may be disposed inboard to a larger extent than shown in Figures 9A and 9B, respectively. In such forms, the grasping areas 985 and 995 would form the outermost portions of the wings 760 and 770.

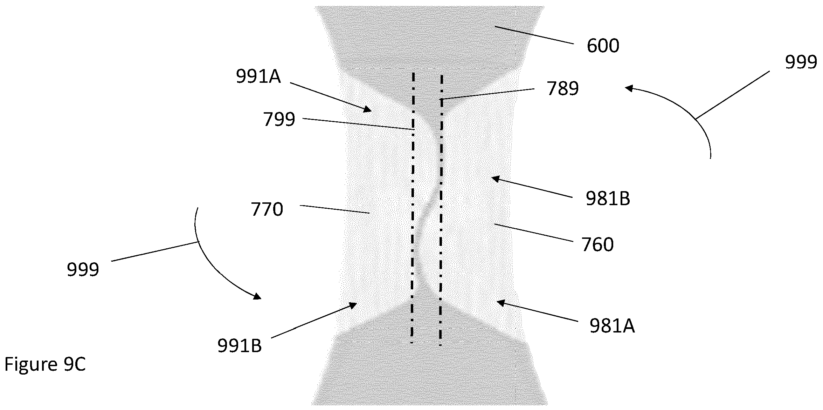

As noted previously, the surface area of the first coverage area and the second coverage area should be similar. In some forms, the ratio between the first coverage area and the second coverage area can be about 1 to 1.48, about 1 to 0.52, or most preferably about 1 to 1, specifically reciting all values within these ranges and any ranges created thereby. Where the surface areas of the first coverage area and the second coverage area are too disparate, premature detachment can occur. In Figure 9C, a moment 999 is shown being applied to the feminine pad. If the ratio of surface area of the first coverage area 981A to the second coverage area 981B for wing 760 is not within the range described previously, the moment 999 can cause premature detachment of the wing 760 - particularly in the second coverage area 981B. This problem may be exacerbated where the ratio of surface area of the first coverage area 991A and the second coverage area 991B of wing 770 are also outside of the ranges described previously.

### Feminine Pad Construction

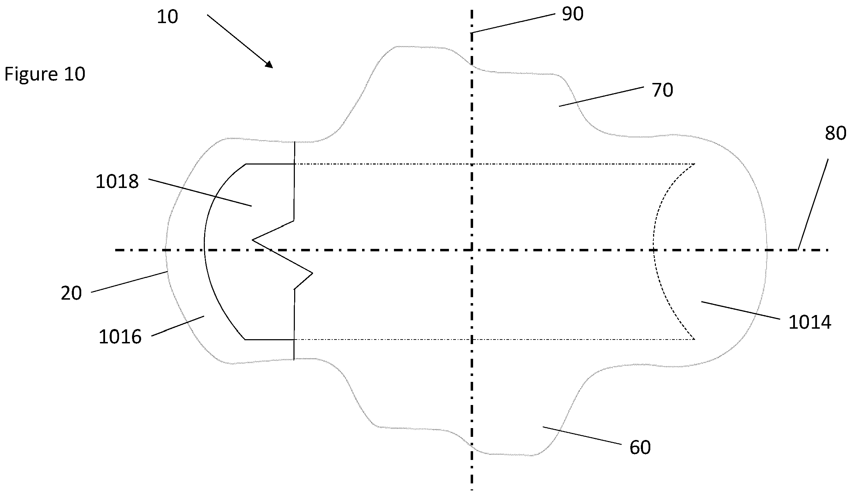

Referring to Figure 10, the feminine pad 110 is shown. As noted previously, the feminine pad 10 comprises the chassis 20 and wings extending outboard of the chassis 20. The chassis 20 comprises a liquid permeable topsheet 1014, a liquid impermeable, or substantially liquid impermeable, backsheet 1016, and an absorbent core 1018 positioned intermediate the topsheet 1014 and the backsheet 1016. The wings 60 and 70 may be joined to the topsheet 1014, the backsheet 1016, and/or the absorbent core 1018. The sanitary napkin 1810 may also be provided with additional features commonly found in sanitary napkins as is known in the art. In some forms of the present invention, the wings may be provided with zones of extensibility as described in U.S. Patent No. 5,972,806.

Any suitable absorbent core known in the art may be utilized. The absorbent core 1018 may be any absorbent member which is generally compressible, conformable, non-irritating to the wearer's skin, and capable of absorbing and retaining liquids such as urine, menses, and/or other body exudates. The absorbent core 1018 may be manufactured from a wide variety of liquid-absorbent materials commonly used in disposable absorbent articles such as comminuted wood pulp which is generally referred to as airfelt. The absorbent core 1018 may comprise superabsorbent polymers (SAP) and less than 15%, less than 10%, less than 5%, less than 3%, or less than 1% of airfelt, or be completely free of airfelt. Examples of other suitable absorbent materials comprise creped cellulose wadding, meltblown polymers including coform, chemically stiffened, modified or cross-linked cellulosic fibers, tissue including tissue wraps and tissue laminates, absorbent foams, absorbent sponges, superabsorbent polymers, absorbent gelling materials, or any equivalent material or combinations of materials.

The configuration and construction of the absorbent core 1018 may vary (e.g., the absorbent core may have varying caliper zones, a hydrophilic gradient, a superabsorbent gradient, or lower average density and lower average basis weight acquisition zones; or may comprise one or more layers or structures). In some forms, the absorbent core 1018 may comprise one or more channels, such as two, three, four, five, or six channels.

The absorbent core 1018 of the present disclosure may comprise one or more adhesives, for example, to help immobilize the SAP or other absorbent materials within a core wrap and/or to ensure integrity of the core wrap, in particular when the core wrap is made of two or more substrates. The core wrap may extend to a larger area than required for containing the absorbent material(s) within.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen.

Other forms and more details regarding channels and pockets that are free of, or substantially free of absorbent materials, such as SAP, within absorbent cores are discussed in greater detail in U.S. Patent Application Publication Nos. 2014/0163500, 2014/0163506, and 2014/0163511, all published on June 12, 2014.

Other suitable materials for use in absorbent cores comprise open celled foams or pieces thereof. The use of foams in absorbent cores is described in additional detail in U.S. Patent Nos. 6,410,820; 6,107,356; 6,204,298; 6,207,724; 6,444,716; 8,211,078, and 8,702,668.

In some forms, the absorbent core structure may comprise a heterogeneous mass layer or may utilize methods or parameters such as those described in US patent application nos. 14/715,984, filed May 19, 2015; US Patent Application No. 14/750,399, June 25, 2015; US Patent Application No. 14/751,969 filed June 26, 2015; US Patent Application No. 15/078,132 filed March 23, 2016; US Patent Application No. 14/750,596 filed June 25, 2015; US Patent Application No. 15/084,902 filed March 30, 2016; US Patent Application No. 15/343,989 filed November 4, 2016; US Patent Application No. 15/344,273 filed November 4, 2016; US Patent Application No. 15/344,294 filed November 4, 2016; US Patent Application No. 14/704,110 filed May 5, 2015; US Patent Application No. 15/194,894 filed June 28, 2016; US Patent Application No. 15/344,050 filed November 4, 2016; US Patent Application No. 15/344,117 filed November 4, 2016; US Patent Application No. 15/344,177 filed November 4, 2016; US Patent Application No. 15/344,198 filed November 4, 2016; US Patent Application No. 15/344,221 filed November 4, 2016; US Patent Application No. 15/344,239 filed November 4, 2016; US Patent Application No. 15/344,255 filed November 4, 2016; US Patent Application No. 15/464,733 filed November 4, 2016; US Provisional Patent Application No. 62/437,208 filed December 21, 2016; US Provisional Patent Application No. 62/437,225 filed December 21, 2016; US Provisional Patent Application No. 62/437,241 filed December 21, 2016; or US Provisional Patent Application No. 62/437,259 filed December 21, 2016. The heterogeneous mass layer has a depth, a width, and a height.

In some forms, a combination of absorbent core materials may be utilized. For example, forms are contemplated where a first layer of an absorbent core comprises a foam material or pieces thereof as described previously and a second layer of an absorbent core comprises an airlaid material. Such combinations are described in U.S. Patent Publication No. 2014/0336606 and U.S. Patent No. 9,649,228.

The feminine pad 1010 may comprise additional layers between the topsheet 1014 and the absorbent core 1018. For example, the feminine pad 1010 may comprise a secondary topsheet and/or an acquisition layer positioned between the topsheet 1014 and the absorbent core 1018. One suitable example of a secondary topsheet is with regard to a spunlace nonwoven. Suitable spunlace nonwovens are discussed in additional detail in U.S. Patent Publication No. 2015/0351976. In some forms, the secondary topsheet may comprise superabsorbent similar to the superabsorbent in the absorbent core or different than the absorbent core.

The backsheet can comprise a liquid impervious film. The backsheet can be impervious to liquids (e.g., body fluids) and can be typically manufactured from a thin plastic film. However, typically the backsheet can permit vapours to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². However, it should be noted that other flexible liquid impervious materials may be used as the backsheet. Herein, "flexible" refers to materials which are compliant and which will readily conform to the general shape and contours of the wearer's body.

The backsheet can be typically positioned adjacent an outer-facing surface of the absorbent core and can be joined thereto by any suitable attachment device known in the art. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment device may include heat bonds, thermal fusion bonds, pressure bonds, ultrasonic bonds, dynamic mechanical bonds, or any other suitable attachment device or combinations of these attachment devices. The backsheet may be additionally secured to the topsheet by any of the above-cited attachment devices / methods.

The topsheet 1014 is positioned adjacent a body-facing surface of the feminine pad 1010. The topsheet 1014 may be joined to the backsheet 1016 by attachment methods (not shown) such as those well known in the art. The topsheet 1014 and the backsheet 1016 may be joined directly to each other in the feminine pad periphery and may be indirectly joined together by directly joining them to the absorbent core 1018 by any suitable attachment method.

The topsheet 1014 may be compliant, soft feeling, and non-irritating to the wearer's skin. Further, the topsheet 1014 may be liquid pervious permitting liquids (e.g., urine, menses) to readily penetrate through its thickness. Some suitable examples of topsheet materials include films, nonwovens, laminate structures including film / nonwoven layers, film / film layers, and nonwoven / nonwoven layers. Other exemplary topsheet materials and designs are disclosed in U.S. Patent Application Publication Nos. 2016/0129661, 2016/0167334, and 2016/0278986.

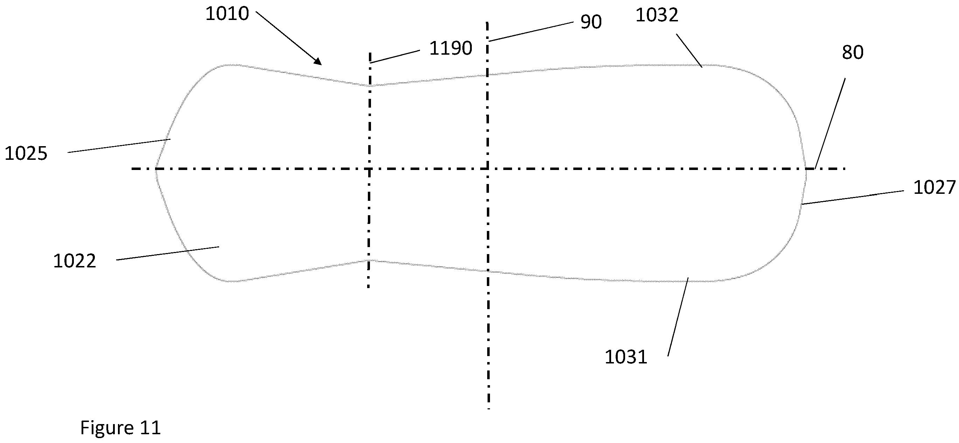

As noted previously, the feminine pad 1010 may comprise a secondary topsheet 1022 or an acquisition layer disposed between the topsheet 1014 and the absorbent core 1018 as shown in Figure 11. The secondary topsheet 1022 may comprise a first end 1025 and an opposing second end 1027 and a pair of longitudinally opposing side edges 1031 and 1032 connecting the first end 1025 and the second end 1027. As shown, the secondary topsheet 1022 may be asymmetrical about the lateral centreline 90.

The secondary topsheet 1022 may additionally comprise a variable width. For example adjacent the first end 1025, the secondary topsheet may have a first width. The width may decrease toward a flex axis 1190. The flex axis 1190 may comprise the smallest width of the secondary topsheet 1022 and may be disposed on a first side of the lateral centreline 90.

The flex axis 1190 can provide the feminine pad with reduced stiffness in the area of the pad adjacent the flex axis 1190. Because the absorbent core and secondary topsheet are typically the thickest materials in the feminine pad, they can also be the stiffest. So, the creation of a flex axis 1190 can provide the feminine pad with the ability to conform to the body of the wearer in the area of the flex axis 1190. And conformance of the feminine pads of the present disclosure to a user's body can provide for a more comfortable use experience for the user.

The wings of the feminine hygiene pads of the present disclosure may be integrally formed as part of the topsheet. In some forms, the wings may be integrally formed as part of the backsheet. In some forms, the wings may be integrally formed as part of the topsheet and the backsheet. In some forms, the wings may be integrally formed with additional layers - described herein - of the feminine hygiene pad. Yet in other forms, the wings may be formed discretely and joined to the chassis.

### Examples

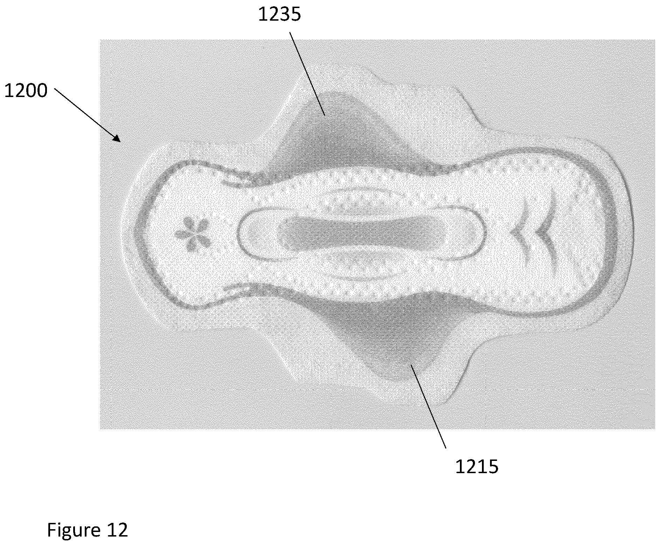

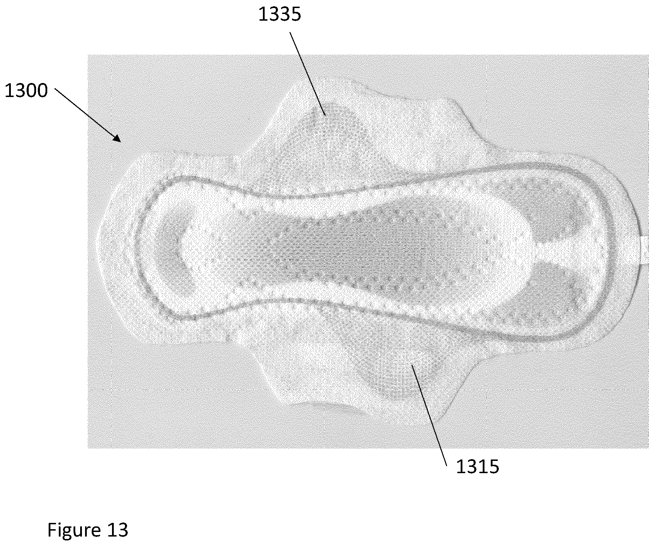

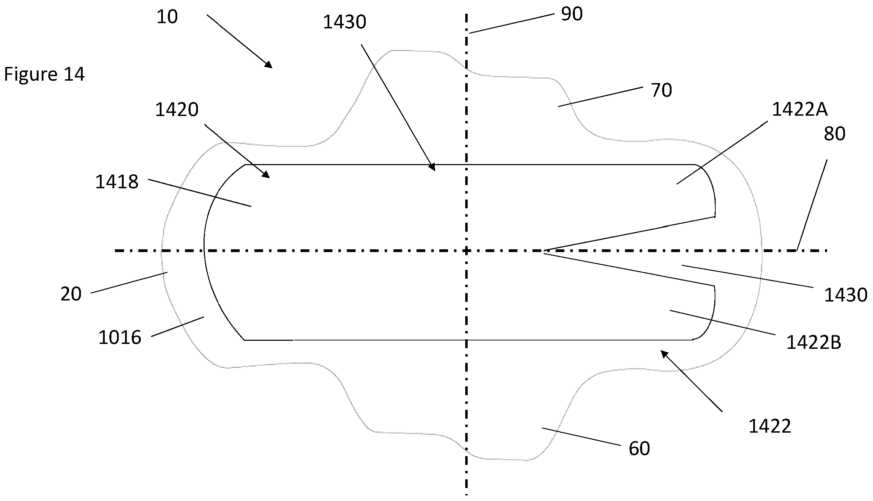

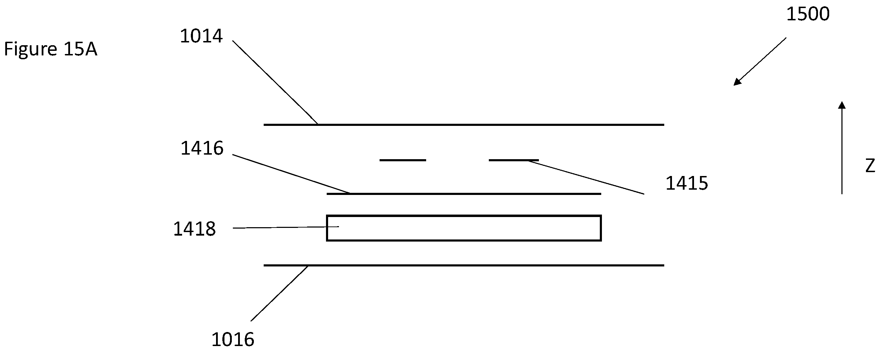

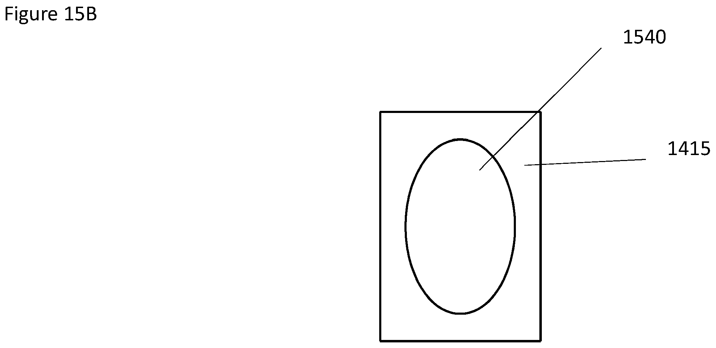

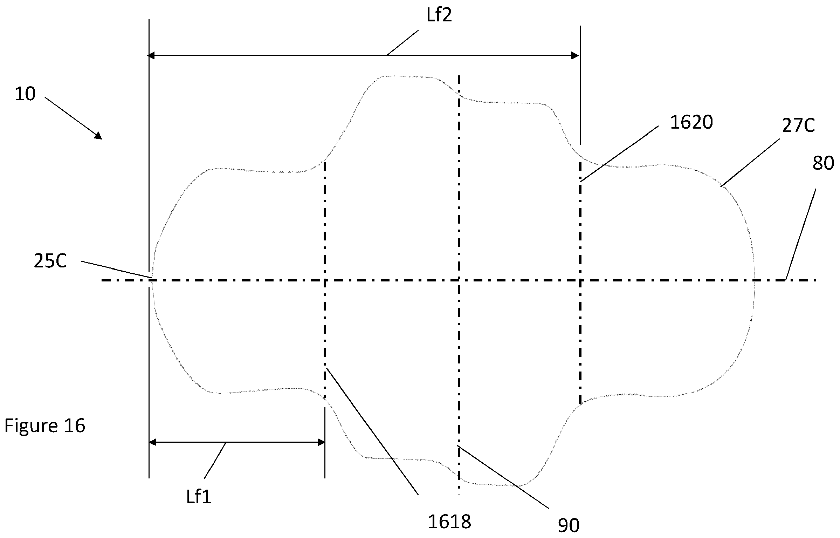

Examples of feminine pads constructed in accordance with the present disclosure are provided with regard to Figures 12 and 13. In Figure 12, a feminine pad 1200 is shown comprising a first visual signal 1215 and a second visual signal 1235. In Figure 13, a feminine pad 1300 is shown comprise a first visual signal 1315 and a second visual signal 1335. Various measurements were taken in accordance with the descriptions in the specification. The various measurements are provided below in Tables 1 and 2. For the sake of clarity, the samples identified as 1200 (1) are associated with a first wing of the pad 1200 in Figure 12, and 1200 (2) are associated with the second wing of the pad 1200 in Figure 12. Similarly, for pad 1300, the measurements are labeled 1300 (1) for the first wing of the pad 1300 in Figure 13 and 1300 (2) for the second wing of the pad 1300 in Figure 13.

**Table 1**

| Pad | Wf (mm) | Ws (mm) | Wing Length (mm) | Distal Zone Area (mm²) | Proximal Zone Area (mm²) |
|---|---|---|---|---|---|
| 1200 (1) | 85.6 | 87.4 | 115.8 | 1374.2 | 1122.6 |
| 1200 (2) | | | 116.8 | 1341.9 | 1025.8 |
| 1300 (1) | 85.7 | 87.6 | 120.8 | 1361.0 | 1239.4 |
| 1300 (2) | | | 122.3 | 1449.3 | 1125.5 |

**Table 2**

| Pad | Visual Signal Area - Distal (mm²) | Visual Signal Area - Proximal (mm²) |
|---|---|---|
| 1200 (1) | 677.4 | 57.4 |
| 1200 (2) | 587.7 | 216.1 |
| 1300(1) | 676.2 | 113.0 |
| 1300 (2) | 677.4 | 18.3 |

### Test Methods

### Visual Signal Area Measurement Method

The intent of the Visual Signal Area measurement is to determine the amount of printed color and/or emboss area within a respective zone of an absorbent article wing. The printed color area and emboss area measurements are obtained from article images acquired using a flatbed scanner. The scanner is capable of scanning in reflectance mode at a resolution of 500 dpi and 24 bit color (a suitable scanner is an Epson Perfection V750 Pro from Epson America Inc., Long Beach CA or equivalent). The scanner is interfaced with a computer running an image analysis program (a suitable program is ImageJ v. 1.50 or equivalent, National Institute of Health, USA). The article images are distance calibrated against an acquired image of a ruler certified by NIST. The resulting image is then analyzed using the image analysis program to identify the boundaries of the printed color and emboss regions and calculate the Visual Signal Area.

### Image Acquisition

The ruler is placed on the center of the scanner bed, oriented parallel to the sides of the scanner glass, and the scanner lid is closed. A calibration image of the ruler is acquired in reflectance mode at a resolution of 500 dpi (approximately 19.7 pixels per mm) and 24 bit color. The calibration image is saved as an uncompressed TIFF format file. The scanner lid is opened and the ruler removed. After obtaining the calibration image, all articles are scanned under the same conditions and measured based on the same calibration file. Next, the article is placed onto the center of the scanner bed, lying flat, with the color printed surface of the article facing the scanner's glass surface. The article is covered with a white background (in this test method white is defined as having L* > 94, -2 < a* < 2, and -2 < b* < 2) and the scanner lid is closed. A scanned image of the article is acquired and saved as an uncompressed TIFF format file. If the size of the article exceeds the available scanning area, multiple scans are obtained covering the entire article and stitched together into a single image for analysis. A total of five substantially similar replicate articles are scanned in like fashion.

### Printed Color and Emboss Area Measurement

The calibration image file is opened in the image analysis program and a linear distance calibration is performed using the imaged ruler. This distance calibration scale is applied to all subsequent article images prior to analysis. An article image is opened in the image analysis program and the distance scale is set. Using the image analysis program, the boundaries of the respective zones of the article wing, within which the printed color area is to be measured, is identified and defined as described within the specification. Using the image analysis program, the boundaries of any printed color regions within the respective zones are identified and defined. Identification of color region boundaries is performed with the intent of defining them as they would be discerned by a human viewer under standard lighting conditions with the unaided eye if being viewed face on in a flat configuration at approximately an arm's length distance. In like fashion, the boundaries of any emboss regions within the respective zones are identified and defined. The area of each of the individual printed color and emboss regions are calculated within the respective zones to the nearest 0.1 mm². Any area containing both printed color and emboss regions is only calculated once. The total area of printed color and emboss regions is calculated by summing up the areas of the individual printed color and emboss regions. This value is recorded as the Visual Signal Area to the nearest 0.1 mm². In like fashion, the remaining four specimen images are analyzed. The average Visual Signal Area is calculated and reported to the nearest 0.1 mm² for the five replicates.

### Linear Distances

Linear distances may be measured by any appropriate instrument that is calibrated and capable of a measurement to the nearest 0.1 mm. Area measurements are made using the projected area of the article, as viewed orthogonally to the plane of the article length and width, in square millimeters to the nearest 0.1 mm².

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A feminine hygiene article (10) having a chassis (20) comprising a topsheet, a backsheet, and an absorbent core disposed between the topsheet and the backsheet, a longitudinal centerline (80) and a lateral centerline (90) perpendicular to the longitudinal centerline, the feminine hygiene article further comprising:
a first wing (60, 360, 460, 660, 760) extending laterally outboard of the chassis having a first leading edge (261, 361, 461) extending outboard of the chassis, a first trailing edge (265, 365, 465) extending outboard of the chassis and one or more edges connecting the first leading edge and the first trailing edge, the first wing further comprising a first proximal zone (281, 381, 481, 681) and a first distal zone (285, 385, 485, 685) and a first bisecting line (287, 387, 487, 787) forming at least a portion of a boundary between the first distal zone and the first proximal zone, and wherein the first wing is asymmetric about the first bisecting line;
a second wing (70, 370, 470, 670, 770) extending laterally outboard of the chassis having a second leading edge (271, 371, 471) extending outboard of the chassis, a second trailing edge (275, 375, 475) extending outboard of the chassis and one or more edges connecting the second leading edge and the second trailing edge, the second wing further comprising a second proximal zone (295, 395, 495, 695) and a second distal zone (291, 391, 491, 691) and a second bisecting line (297, 397, 497, 797) forming at least a portion of a boundary between the second distal zone and the second proximal zone, and wherein the second wing is asymmetric about the second bisecting line.
a first visual signal disposed in the first distal zone; and
a second visual signal disposed in the second distal zone;
wherein the distal zones comprise a higher amount of visual signaling than their proximal zone counterparts, and wherein the first distal zone and the second distal zone are longitudinally offset from one another;
the feminine hygiene article further comprising a first end (25) and an opposing second end (27), wherein the first leading edge is more proximal to the first end than the first trailing edge, and wherein the second leading edge is more proximal to the first end than the second trailing edge; and wherein the first leading edge (461) extends toward the second end of the feminine pad and transitions into a first distal edge (464); and the first distal edge (464) transitions into the first trailing edge (465) which joins the first distal edge (464) and the chassis (20).

2. The feminine hygiene article of claim 1, wherein the first visual signal extends into the first proximal zone and the second visual signal extends into the second proximal zone.

3. The feminine hygiene article of any of claims 1 or 2, wherein the first visual signal and the second visual signal comprise a print signal.

4. The feminine hygiene article of any of claims 1 or 2, wherein the first visual signal and the second visual signal comprise embossing.

5. The feminine hygiene article any of the preceding claims, wherein the first distal zone is disposed on one side of the first bisecting line of the feminine hygiene article and the second distal zone is disposed on a second side of the second bisecting line.

6. The feminine hygiene article of claim 1, wherein the first distal zone is more proximal to the second end than the first proximal zone.

7. The feminine hygiene article of any of claims 1 or 6, wherein the second distal zone is more proximal to the first end than the second proximal zone.

8. The feminine hygiene article any of claims 1, 6 or 7, wherein the second trailing edge (475) extends toward the first end of the feminine pad and transitions into a second distal edge (472); and the second distal edge (472) transitions into the second leading edge (471) which joins the second distal edge (472) and the chassis (20).

9. The feminine hygiene article of any of claims 1, 6, 7 or 8, wherein the first end comprises a pair of longitudinal sides (25A, 25B) and a width Wf generally parallel to the lateral centerline, wherein the width Wf represents the widest portion of the feminine pad in the first end, and wherein the width of the first end narrows toward the first leading edge and the second leading edge.

10. The feminine hygiene article of any of the preceding claims, wherein the first visual signal is disposed on a first surface (501) of the first wing, and wherein the second visual signal is disposed on a first surface (503) of the second wing.

## Patentansprüche

1. Damenhygieneartikel (10), der eine Grundeinheit (20) aufweist, umfassend eine Oberschicht, eine Unterschicht und einen Absorptionskern, der zwischen der Oberschicht und der Unterschicht angeordnet ist, eine Mittellinie in Längsrichtung (80) und eine Querachse (90) lotrecht zu der Mittellinie in Längsrichtung, der Damenhygieneartikel ferner umfassend:
einen ersten Flügel (60, 360, 460, 660, 760), der sich seitlich außerhalb der Grundeinheit erstreckt, der einen ersten vorderen Rand (261, 361, 461), der sich außerhalb der Grundeinheit erstreckt, einen ersten hinteren Rand (265, 365, 465), der sich außerhalb der Grundeinheit erstreckt, und einen oder mehrere Ränder aufweist, die den ersten vorderen Rand und den ersten hinteren Rand verbinden, der erste Flügel ferner umfassend einen ersten nahe gelegenen Bereich (281, 381, 481, 681) und einen ersten distalen Bereich (285, 385, 485, 685) und eine erste Halbierungslinie (287, 387, 487, 787), die wenigstens einen Teil einer Grenze zwischen dem ersten distalen Bereich und dem ersten nahe gelegenen Bereich ausbildet, und wobei der erste Flügel um die erste Halbierungslinie herum asymmetrisch ist;
einen zweiten Flügel (70, 370, 470, 670, 770), der sich seitlich außerhalb der Grundeinheit erstreckt, der einen zweiten vorderen Rand (271, 371, 471), der sich außerhalb der Grundeinheit erstreckt, einen zweiten hinteren Rand (275, 375, 475), der sich außerhalb der Grundeinheit erstreckt, und einen oder mehrere Ränder aufweist, die den zweiten vorderen Rand und den zweiten hinteren Rand verbinden, der zweite Flügel ferner umfassend einen zweiten nahe gelegenen Bereich (295, 395, 495, 695) und einen zweiten distalen Bereich (291, 391, 491, 691) und eine zweite Halbierungslinie (297, 397, 497, 797), die wenigstens einen Teil einer Grenze zwischen dem zweiten distalen Bereich und dem zweiten nahe gelegenen Bereich ausbildet, und wobei der zweite Flügel um die zweite Halbierungslinie herum asymmetrisch ist.
ein erstes visuelles Signal, das in dem ersten distalen Bereich angeordnet ist; und
ein zweites visuelles Signal, das in dem zweiten distalen Bereich angeordnet ist;
wobei die distalen Bereiche eine größere Menge an visueller Signalisierung als ihre nahe gelegenen Bereichsgegenstücke umfassen und wobei der erste distale Bereich und der zweite distale Bereich in Längsrichtung voneinander versetzt sind;
der Damenhygieneartikel ferner umfassend ein erstes Ende (25) und ein entgegengesetztes zweites Ende (27), wobei der erste vordere Rand näher gelegen zu dem ersten Ende als der erste hintere Rand ist und wobei der zweite vordere Rand näher gelegen zu dem ersten Ende als der zweite hintere Rand ist; und
wobei der erste vordere Rand (461) sich zu dem zweiten Ende der Dameneinlage hin erstreckt und in einen ersten distalen Rand (464) übergeht; und der erste distale Rand (464) in den ersten hinteren Rand (465) übergeht, der den ersten distalen Rand (464) und die Grundeinheit (20) zusammenfügt.

2. Damenhygieneartikel nach Anspruch 1, wobei sich das erste visuelle Signal in den ersten nahe gelegenen Bereich erstreckt und sich das zweite visuelle Signal in den zweiten nahe gelegenen Bereich erstreckt.

3. Damenhygieneartikel nach einem der Ansprüche 1 oder 2, wobei das erste visuelle Signal und das zweite visuelle Signal ein Drucksignal umfassen.

4. Damenhygieneartikel nach einem der Ansprüche 1 oder 2, wobei das erste visuelle Signal und das zweite visuelle Signal eine Prägung umfassen.

5. Damenhygieneartikel nach einem der vorstehenden Ansprüche, wobei der erste distale Bereich auf einer Seite der ersten Halbierungslinie des Damenhygieneartikels angeordnet ist und der zweite distale Bereich auf einer zweiten Seite der zweiten Halbierungslinie angeordnet ist.

6. Damenhygieneartikel nach Anspruch 1, wobei der erste distale Bereich näher gelegen zu dem zweiten Ende als der erste nahe gelegene Bereich ist.

7. Damenhygieneartikel nach einem der Ansprüche 1 bis 6, wobei der zweite distale Bereich näher gelegen zu dem ersten Ende als der zweite nahe gelegene Bereich ist.

8. Damenhygieneartikel nach einem der Ansprüche 1, 6 oder 7, wobei sich der zweite hintere Rand (475) zu dem ersten Ende der Dameneinlage hin erstreckt und in einen zweiten distalen Rand (472) übergeht; und der zweite distale Rand (472) in den zweiten vorderen Rand (471) übergeht, der den zweiten distalen Rand (472) und die Grundeinheit (20) zusammenfügt.

9. Damenhygieneartikel nach einem der Ansprüche 1, 6, 7 oder 8, wobei das erste Ende ein Paar von Längsseiten (25A, 25B) und eine Breite Wf im Allgemeinen parallel zu der Querachse umfasst, wobei die Breite Wf den breitesten Teil der Dameneinlage in dem ersten Ende darstellt und wobei sich die Breite des ersten Endes zu dem ersten vorderen Rand und dem zweiten vorderen Rand hin verjüngt.

10. Damenhygieneartikel nach einem der vorstehenden Ansprüche, wobei das erste visuelle Signal auf einer ersten Oberfläche (501) des ersten Flügels angeordnet ist und wobei das zweite visuelle Signal auf einer ersten Oberfläche (503) des zweiten Flügels angeordnet ist.

## Revendications

1. Article d'hygiène féminine (10) présentant un châssis (20) comprenant une feuille de dessus, une feuille de fond, et une âme absorbante disposée entre la feuille de dessus et la feuille de fond, une ligne médiane longitudinale (80) et une ligne médiane latérale (90) perpendiculaire à la ligne médiane longitudinale, l'article d'hygiène féminine comprenant en outre :
une première aile (60, 360, 460, 660, 760) s'étendant dans la direction latérale à l'extérieur du châssis ayant un premier bord d'attaque (261, 361, 461) s'étendant à l'extérieur du châssis, un premier bord arrière (265, 365, 465) s'étendant à l'extérieur du châssis et un ou plusieurs bords reliant le premier bord d'attaque et le premier bord arrière, la première aile comprenant en outre une première zone proximale (281, 381, 481, 681) et une première zone distale (285, 385, 485, 685) et une première ligne bissectrice (287, 387, 487, 787) formant au moins une partie d'une limite entre la première zone distale et la première zone proximale, et dans lequel la première aile est asymétrique autour de la première ligne bissectrice ;
une seconde aile (70, 370, 470, 670, 770) s'étendant dans la direction latérale à l'extérieur du châssis ayant un second bord d'attaque (271, 371, 471) s'étendant à l'extérieur du châssis, un second bord arrière (275, 375, 475) s'étendant à l'extérieur du châssis et un ou plusieurs bords reliant le second bord d'attaque et le second bord arrière, la seconde aile comprenant en outre une seconde zone proximale (295, 395, 495, 695) et une seconde zone distale (291, 391, 491, 691) et une seconde ligne bissectrice (297, 397, 497, 797) formant au moins une partie d'une limite entre la seconde zone distale et la seconde zone proximale, et dans lequel la seconde aile est asymétrique autour de la seconde ligne bissectrice ;
un premier signal visuel disposé dans la première zone distale ; et
un second signal visuel disposé dans la seconde zone distale ;
dans lequel les zones distales comprennent une quantité supérieure de signalisation visuelle que leurs homologues zones proximales, et dans lequel la première zone distale et la seconde zone distale sont décalées l'une par rapport à l'autre dans la direction longitudinale ;
l'article d'hygiène féminine comprenant en outre une première extrémité (25) et une seconde extrémité opposée (27), dans lequel le premier bord d'attaque est plus proximal à la première extrémité que le premier bord arrière, et dans lequel le second bord d'attaque est plus proximal à la première extrémité que le second bord arrière ; et
dans lequel le premier bord d'attaque (461) s'étend vers la seconde extrémité du coussinet féminin et passe à un premier bord distal (464) ; et le premier bord distal (464) passe au premier bord de fuite (465) qui joint le premier bord distal (464) et le châssis (20).

2. Article d'hygiène féminine selon la revendication 1, dans lequel le premier signal visuel s'étend dans la première zone proximale et le second signal visuel s'étend dans la seconde zone proximale.

3. Article d'hygiène féminine selon l'une quelconque des revendications 1 à 2, dans lequel le premier signal visuel et le second signal visuel comprennent un signal d'impression.

4. Article d'hygiène féminine selon l'une quelconque des revendications 1 à 2, dans lequel le premier signal visuel et le second signal visuel comprennent un gaufrage.

5. Article d'hygiène féminine selon l'une quelconque des revendications précédentes, dans lequel la première zone distale est disposée sur un côté de la première ligne bissectrice de l'article d'hygiène féminine et la seconde zone distale est disposée sur un second côté de la seconde ligne bissectrice.

6. Article d'hygiène féminine selon la revendication 1, dans lequel la première zone distale est plus proximale de la seconde extrémité que la première zone proximale.

7. Article d'hygiène féminine selon l'une quelconque des revendications 1 à 6, dans lequel la seconde zone distale est plus proximale de la première extrémité que la seconde zone distale.

8. Article d'hygiène féminine selon l'une quelconque des revendications 1, 6 ou 7, dans lequel le second bord de fuite (475) s'étend vers la première extrémité du coussinet féminin et passe à un second bord distal (472) ; et le second bord distal (472) passe au second front avant (471) qui joint le second bord distal (472) et le châssis (20).

9. Article d'hygiène féminine selon l'une quelconque des revendications 1, 6, 7 ou 8, dans lequel la première extrémité comprend une paire de côtés longitudinaux (25A, 25B) et une largeur Wf généralement parallèle à la ligne médiane latérale, dans lequel la largeur Wf représente la partie la plus large du tampon féminin dans la première extrémité, et dans lequel la largeur de la première extrémité se rétrécit vers le premier bord d'attaque et le second bord d'attaque.

10. Article d'hygiène féminine selon l'une quelconque des revendications précédentes, dans lequel le premier signal visuel est disposé sur une première surface (501) de la première aile, et dans lequel le second signal visuel est disposé sur une première surface (503) de la seconde aile.
